(19) Europäisches Patentamt / European Patent Office / Office européen des brevets

(11) **EP 4 310 858 A1**

(12) **EUROPEAN PATENT APPLICATION**

(43) Date of publication:
**24.01.2024 Bulletin 2024/04**

(21) Application number: **22185439.1**

(22) Date of filing: **18.07.2022**

(51) International Patent Classification (IPC):
*G16H 50/30* (2018.01)   *G16H 50/20* (2018.01)
*G16B 5/10* (2019.01)   *G16B 20/40* (2019.01)
*G16B 25/10* (2019.01)   *G16B 40/20* (2019.01)
*G16B 40/30* (2019.01)

(52) Cooperative Patent Classification (CPC):
**G16B 5/10; G16B 20/40; G16B 25/10; G16B 40/20; G16B 40/30; G16H 50/20; G16H 50/30**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Leibniz-Institut Für Altersforschung**
  **07745 Jena (DE)**

• **Scuola Normale Superiore**
  **56126 Pisa (IT)**

(72) Inventors:
• **Cellerino, Alessandro**
  **Pisa (IT)**
• **Ferrari, Elisa**
  **Pisa (IT)**

(74) Representative: **Dennemeyer & Associates S.A.**
  **Landaubogen 1-3**
  **81373 München (DE)**

(54) **A METHOD TO PREDICT LIFESPAN AND HEALTHSPAN**

(57) The invention relates to a method for predicting the lifespan of an individual, comprising i. providing biological input data of the subject, ii. providing a list of confounding variables, iii. predicting an age-related mortality or disease, preferably the time to death and/or the mortality risk and/or the risk of age-related disease, for the subject by analyzing the data with an algorithm, wherein the algorithm comprises a neural network, which is trained on at least one reference dataset comprising biological data of at least one reference subject by applying: a) a selector layer to filter input data of i., and b) an adversarial learning framework, that removes from the input data the information related to the confounding variables, wherein preferably the adversarial learning framework, is a neural network comprising three elements: a feature extractor (FE), a predictor (P), and a confounder predictor (C). The invention further relates to a device or system comprising means for carrying out the steps of the method according to the invention, a computer program [product] and a computer-readable storage medium.

Fig. 3

**Description**

The invention is in the field of methods for predicting biological age or life expectancy. Aspects of the invention are in the field of computer-implemented approaches to predict lifespan, biological age or life expectancy.

The invention relates to a method for predicting the lifespan of an individual. In embodiments of the invention, the method comprises i. providing biological input data of the subject, ii. providing a list of confounding variables, iii. predicting an age-related mortality or disease, such as time to death, mortality risk and/or a risk of age-related disease, for the subject by analyzing the data with an algorithm, wherein the algorithm comprises a neural network, which is trained on at least one reference dataset comprising biological data of multiple reference subjects by applying: a) a selector layer to filter input data of i., and b) an adversarial learning framework, that removes from the input data the information related to the confounding variables of ii, wherein preferably the adversarial learning framework, is a neural network comprising three elements: a feature extractor (FE), a predictor (P), and a confounder predictor (C).

The invention further relates to a device or system comprising means for carrying out the steps of the method according to the invention, a computer program and computer program product and a computer-readable storage medium.

**BACKGROUND OF THE INVENTION**

The capability to predict individual biological age and life expectancy from genetic and epigenetic data is attracting growing interest in the field of aging research. Since nearly two decades, there has been a growing interest in the development of age predictors based on biological information, mainly DNA methylation or gene expression data, the so-called epigenetic/transcriptomic clocks. The appeal of these predictors relies in the fact that algorithms trained to predict the chronological age of various individuals based on their biological data, when applied to a new set of subjects, estimate individual age with an error that is considered to reflect the individual differences in aging speed. In other words, subjects whose age is overestimated were reported to score worse on physiological measures of the aging process and risk factors for age-related diseases, i.e., they have aged faster, and those whose age is underestimated were reported to score better on the same measures.

One primary practical interest in these age predictor lies in the possibility to use them as surrogate markers of lifespan to assess the effects of interventions aimed at manipulating human aging, i.e., interventions that reduce "biological age" are expected to prolong lifespan and promote health-span in humans.

The use of these predictors is based on the assumption that the prediction error has a biological meaning. Although this correlation has sometimes been proven to exist, the prediction error cannot be considered a reliable measure of aging at the individual level. In fact, machine learning based algorithms can be misled by multiple factors and can notoriously commit randomly large errors on data obtained with a data generation process only slightly different from the one used to obtain the training data. In fact, more recent and exhaustive studies on the reliability of some widely used epigenetic clocks show no association between epigenetic clocks and loss in functional capacities and health deterioration assessed longitudinally (Vetter et al., 2022).

Mainly two well-known phenomena can mislead the supervised learning process of a machine learning (or deep learning) algorithm causing its predictions to have apparently unmotivated large errors.

First, a phenomenon called "overfitting", which occurs more frequently when the input data have a high dimensionality. Basically, the high number of input variables requires the model to use a high number of parameters to fit them. The more are the parameters of the algorithm, the more are the degrees of freedom of the fit, which means that it is possible to perfectly fit any data, including noise. Consequently, the high dimensionality of the input data, such as the one of the biological data used to train epigenetic/transcriptomic clocks, increases the chances that the machine learning method may learn random patterns that overfit the training set and does not fit any other data. Direct consequences of overfitting are that the performances are overestimated on the training set and significantly drop when the algorithm is applied to new data and the fact that the learned pattern does not represent any biologically relevant information.

Second, a phenomenon called "confounding effect". The confounding effect occurs because supervised learning tries to minimize the prediction errors made on the training dataset simply looking for correlations among the inputs and their paired outputs. No causal reasoning is included in the algorithm. Consequently, if the distribution of the outputs is correlated to another variable that influences the value of the input data, but is independent from the task of interest, the predictor may learn a pattern based on how this second variable, called confounder, affects the data. Hence, the confounding effect is one of the most deceitful effects in supervised learning. In fact, if the biases present in the training set are equally present in the validation (or test) set, the performance may be misunderstood and often overestimated. These confounding effects can be very detrimental as, despite an apparent reduction in the prediction error with respect to a random guess, the algorithm has not learnt a pattern based on the correct factor (the aging process in the case at hand). This situation occurs frequently when analyzing biological data that depend on a wide number of biological and technical variables (such as gender, environmental conditions and batch effects, etc.).

For instance, in a lifespan prediction study, if different strains of a given species show different life expectancies, the algorithm may learn to distinguish the strains and systematically assign to the members of each strain the mean value of its distribution. In this way, the error on the predictions is smaller than a random guess, but the pattern learned is not related to aging but to genetic differences and cannot be generalized to, for example, outbred individuals. Similarly, when multiple instances of the same individuals (i.e. different samples) are present in the dataset, the predictor may memorize the outcome of each individual and learn to recognize them.

Another well-known confounding effect occurs when input data obtained from different sources (such as tissues, ethnicities or batches) are not equally represented. In this case, the predictor may learn to perfectly fit the data coming from the most represented source, almost ignoring the remaining ones, because this enables a better minimization of the mean error across the entire dataset. This problem is particularly important for epigenetic clock studies in which usually the objective is to find an aging biomarker common to multiple tissues (the so-called pan-tissue clock) but not all of them are equally represented in the dataset.

The aforementioned problems often make the errors committed on the age prediction of the epigenetic/transcriptomic clock independent from real biological differences in the aging speed. If overfitting occurs, the errors committed can be randomly high with no relationship with the underlying biology, simply because the learned pattern fits noise. If some confounding effects occur, the errors are not random and depends on the distribution of the data with respect to the confounding variable.

Thus, measuring the aging speed of a subject based on the error committed in predicting its chronological age is fundamentally incorrect.

A more reliable method to infer the whole aging process, is training an algorithm in such a way that its prediction is the meaningful information, and not the discrepancy between the prediction and the true answer. This would amount to predicting the time to death of an individual. However, this is a very difficult task in humans for two reasons. First, human lifespan is very long. Thus, the interval between collection of the samples and the variable to predict, i.e., age at death, is of several decades. Second, the human lifespan, even of those who died of natural causes, does not depend exclusively on their intrinsic aging speed, but also (and mainly) on sanitary and living conditions, access to healthcare and on whether the pathologies/condition affecting an individual can be treated or not and on other random events such as accidents.

Thus, the most common approach to develop a system to predict time to death consists in two stages: (i) train an algorithm to predict the age of the input data; (ii) use the errors committed on the prediction to estimate the aging speed of each individual and use the latter to further estimate individual time to death.

Besides the fundamentally incorrect adoption of the prediction error as a measure of the aging speed that has been already discussed above, this double stage process suffers also from other flaws. For instance, the algorithm used for the first stage step is usually based on a linear approach (in most of the cases an Elastic Net (EN) is adopted), meaning that it searches a linear combination of the input features that returns the age of the sample. Linear approaches have nearly the same number of internal parameters as the input features, so are less prone to overfitting with respect to non-linear approaches and are easily interpretable: it is always possible to get access to the linear combination used for the prediction. This is very important, because one desirable goal of the development and training of these algorithms consists in identifying a limited set of biological markers (out of the many used as input) that could constitute an aging biomarker that is easy to analyze and monitor across time.

However, even though linear approaches are less prone to overfitting than non-linear approaches, given that the input data have often a very high dimensionality they also suffer from overfitting. Solutions to avoid this phenomenon usually rely in (i) reducing the input data by arbitrary selection of the features to include in the model, that makes the developing of epigenetic/transcriptomic clock dependent on already acquired knowledge on the aging process; (ii) introducing regularization terms in the cost function to reduce the overfitting, as it happens in the EN algorithm. The elastic net, in fact, searches a linear combination of the input features that allows to predict their outputs, minimizing a cost function that contains both the prediction error and regularization terms on the weights L1-norm and L2-norm. In the EN, the L1 regularization encourages the research of a pattern that is simple and sparse (i.e., with a reduced number of features with non-zero weights) respectively, diminishing the probability of overfitting and increasing the interpretability of the model. Furthermore, also the L2 regularization may help to prevent overfitting. In fact, it forces to equally distribute the weights among the non-zero features, which prevents the algorithm to base its predictions on individual features, that may be affected from noise.

Besides these two solutions to reduce overfitting, linear approaches usually do not implement any strategy to avoid, minimize or control neither overfitting nor the second most important problem of supervised learning: the confounding effect. This phenomenon can otherwise be mitigated by either selecting a uniform representation of the data, but this often requires to discard many data, or using a very large training set, but this is rarely possible with real world data.

Furthermore, linear approaches (including EN) can only find patterns based on the linear combination of the input features, while aging is a non-linear phenomenon. In fact, it has been shown that applying non-linear operations to the input data (Meyer, David H., and Björn Schumacher, 2021) or to the outputs of the predictor improve the age estimation. However, the use of non-linear supervised learning approaches such as deep neural networks (DNN) is discouraged by their black-box behavior and because of their large number of internal parameters to optimize, which increases the risk of overfitting.

In literature there are only few examples of predictors incorporating time to death information (Lu et al., 2019; Levine et al., 2018). All of them use a linear approach, that as already said, cannot fully capture the non-linear effects of aging. None of them accounts for confounding effects in the learning phase. In addition, given that collecting human lifespan data requires decades, to make full use of the biological data available of subjects whose age of death was not known, a model specifically designed to handle missing data (e.g. Cox regression) was used. This choice unavoidably limits the accuracy of the predictor. Furthermore, time-to-death data from humans have been acquired only after a certain age, thus these predictors may be reliable only on an aged population.

Summarizing, given the difficulty in obtaining biological data paired with time-to-death information, there has been a growing interest in the development of epigenetic/transcriptomic clocks trained to predict the age of the sample to use their prediction as a measure of individual aging speed. Besides, the fundamentally incorrect usage of prediction errors as the meaningful information of the study, there are some methodological issues, often overlooked, that may reduce the accuracy of the predictions: the overfitting problem and the possible presence of confounding effects. Previous works often tackle the overfitting problem only, by using an EN. However, EN (such as all the linear methods) cannot capture non-linear effects of aging. While non-linear methods such as DNN may overcome this issue, they would be more prone to overfitting, and they are not explainable. Only few works tried to incorporate time-to-death information. Due to the difficulty in getting access to this information, usually methods to handle missing data are used, making the predictions less accurate. Furthermore, the input data only come from an aged population, making the predictor reliable only on this type of population and thus narrowing the potential usefulness of this tool. Finally, these works do not implement any strategy to correct for confounding effects.

In light of these shortcomings of the prior art, there remains a significant need to develop a deep learning-based algorithm to reliably and precisely predict the time-to-death instead of biological age, while at the same time facing the problems of overfitting and confounding effects and being explainable.

## SUMMARY OF THE INVENTION

The abovementioned problems are solved by the features of the independent claims. Preferred embodiments of the present invention are provided by the dependent claims.

One problem underlying the invention is to provide improved or alternative approaches to predict lifespan, biological age or life expectancy. One problem underlying the invention is the need to develop an algorithm that is resilient to the many biological and technical confounders that may mislead the learning process. Another problem underlying the invention is the need to develop a non-linear predictor resilient to overfitting and that can select and make easily accessible a list of a limited number of input features that maximizes predictive performance.

In one aspect, the invention therefore relates to a method for predicting the lifespan of an individual, comprising

    i. providing biological input data of the subject,

    ii. providing a list of confounding variables,

    iii. predicting an age-related mortality or disease of the subject by analyzing the data with an algorithm,

wherein the algorithm comprises a neural network, which is trained on at least one reference dataset comprising biological data of multiple reference subjects by applying:

    a) a selector layer to filter input data of i., and
    b) an adversarial learning framework, that removes from the input data the information related to the confounding variables.

In embodiments the reference dataset comprises biological data of a set of reference subjects paired with their desired output label (i.e., their lifespan or time to death) and the values that the confounding variables assume in these individuals.

In embodiments of the present method in step iii. predicting an age-related mortality or disease comprises predicting the time to death, a mortality risk and/or a risk of age-related disease of the subject.

In embodiments of the present method in step iii. predicting an age-related mortality or disease comprises predicting the time to death and/or a mortality risk of the subject. In embodiments of the present method in step iii. predicting an age-related mortality or disease comprises predicting a risk of age-related disease of the subject.

In one embodiment predicting the lifespan of a subject is predicting of an individual the time to death without predicting the mortality risk and/or the risk of age-related disease.

In some embodiments of the present method predicting the lifespan of a subject comprises predicting the healthspan of a subject, either instead of, or in addition to predicting the lifespan of a subject, wherein the healthspan is the timespan in which the subject is free from chronic disease, (invalidating) disease, disabilities of aging and/or age-related disease.

The problem of the lack of a deep learning-based algorithm to predict the lifespan, the time to death, the mortality risk and/or risk of age-related disease instead of the biological age is overcome by the present invention by the application of a deep learning algorithm that is preferably trained on reference data taken, in some embodiments, from an animal model with a short lifespan, e.g. the fish *Nothobranchius furzeri* whose aging process has proved to share important similarities with that of humans (Irizar et al., 2019).

The use of an animal model under controlled laboratory conditions has the advantage that it facilitates the analysis of biological or biochemical data with associated time to death- (and/or mortality risk- and/or risk of age-related disease-) information in a controlled condition that minimizes external sources of mortality. In other preferred embodiments the reference/training data is derived from human (reference) subjects that were clinically supervised over their whole lifespan, or over the majority or at least for an extended period of their lifespan.

Another shortcoming of prior art methods is that most approaches are forced to pre-select the input data at a preliminary stage to avoid overfitting. However, a data driven selection, such as the one achieved with the selector of the present method, overcomes this problem by avoiding to arbitrarily discard data that is relevant for the prediction.

The invention further addresses other two shortcomings of the prior art: the lack of algorithms designed to be resilient to the biological and technical confounders that may mislead the learning process and the lack of a non-linear predictor resilient to overfitting.

To solve these prior art technical shortcomings, the inventors developed a deep learning architecture comprising in preferred embodiments the use of an adversarial learning framework to address the problem of confounders. The learning process of an algorithm based on adversarial learning basically consists in a trade-off between the minimization in the error prediction on a certain task and its maximization on another task.

In the case of addressing the confounding effect, the present adversarial approach is used in preferred embodiments of the present method to find the best compromise between learning to predict time to death and/or the mortality risk and/or the risk of age-related disease and avoiding learning from confounders.

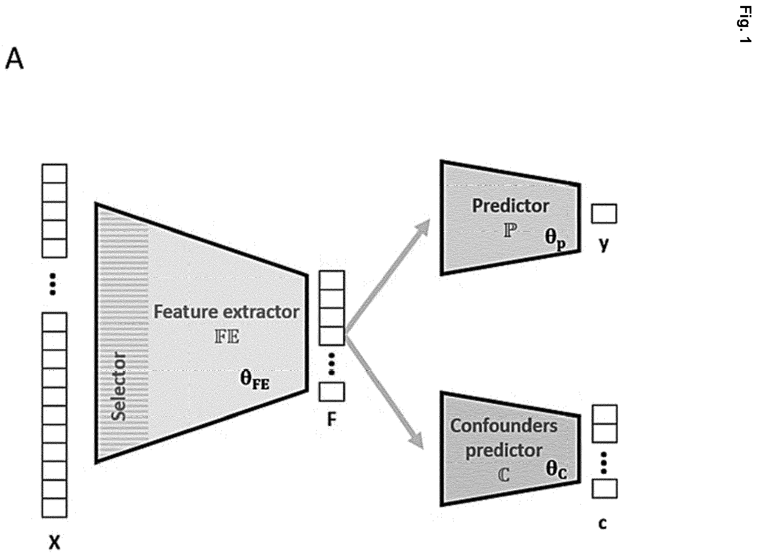

In summary, to achieve the aforementioned advantageous effects over the prior art, the present invention preferably applies the architecture summarized in Fig. 1A, which is composed preferably of three elements/components:

1. A feature extractor (FE), which is responsible for reducing the input information to a vector $\vec{F}$ with a lower dimensionality, whose elements are a non-linear combination of the inputs.

2. A predictor (P), which uses the output of FE to predict an age-related mortality or disease, such as the time to death and/or the mortality risk and/or the risk of age-related disease, paired to the input data X.

3. A confounder predictor (C) that uses the output of FE to predict a vector of categorical and/or continuous confounders, selected by the user, associated to the input data X (such as gender, sample properties, technical variables relevant for acquiring the data, etc.).

Accordingly, in preferred embodiments, the present invention relates to a method for predicting the lifespan of a subject, comprising

i. providing biological data of the subject,

ii. providing a list of confounding variables,

iii. predicting an age-related mortality or disease for the subject by analyzing the data with an algorithm,

wherein the algorithm comprises a (deep) neural network, which is trained on at least one reference dataset comprising biological data of multiple reference subjects by applying:

a) a selector layer to filter input data of i.,
and

b) an adversarial learning framework, that removes from the input data the information related to the confounding variables, and comprising:

    I. a feature extractor (FE),
    II. a predictor (P), and
    III. a confounder predictor (C).

In preferred embodiments, the surprising and advantageous effect of an improved prediction of an individual's age-related mortality or disease, and accordingly of the time to death and/or lifespan and/or healthspan and/or the mortality risk and/or the risk of age-related disease, is achieved by the present method, among other things, through the combination of its features, such as the adversarial learning (framework) and the application of a selector (layer).

In preferred embodiments the adversarial learning setup forces the neural network to select a set of features that maximizes the performance prediction of the desired task, but at the same time minimizes the performance prediction of the possible confounders. Besides preventing learning from possible confounders, this sort of 'tug of war' indirectly reduces overfitting, because it forces the algorithm to find alternative patterns with respect to the one that would simply maximize the performance alone.

The pattern learned by a neural network is notoriously very difficult to extract and analyze. For this reason, preferred embodiments of the present method put a binary feature selector as first layer of the neural network, that assigns a binary weight (0 or 1) to each input data point, e.g., in case of the input data being genomics data, to each gene. This allows to filter the data points, e.g., genes, of interest to predict the output. The selector is also regularized in order to partially control the number of data points, e.g., genes, to select. Thanks to the presence of the binary selector, it is possible to extract the list of data points, e.g., genes, chosen by the algorithm to predict the output. Furthermore, the research of a sparse pattern contributes to avoid overfitting. Accordingly, the introduction of a binary feature selector in the neural net in combination with adversarial learning approach achieves beneficial results.

A neural network can be considered as a sequence of mathematical operations, organized in layers, that processes the input into the output. In embodiments of the present method the weights of each layer are optimized during training in order to minimize the prediction error of the desired task (i.e., the time-to-death prediction) while maximizing the prediction error on the confounding variables. Hence, in embodiments dropout layers can be used to randomly and temporary set some of their weights to zero, such that algorithm is forced to optimize the non-zero weights, which prevents the model from fitting noise in the training data.

In one embodiment the invention relates to a method for predicting the lifespan of an individual, wherein the selector assigns a weight between 0 and 1 to each data variable by multiplying said variable with a number between 0 and 1, wherein a weight is not modified during the gradient calculation but is rounded to the nearest integer during the inference process.

In embodiments of the method according to the present invention a cut-L1 norm regularization is used to encourage the assignment of 0-weights by the selector, wherein a standard L1 norm regularization imposes the deep neural network to minimize the sum of the selector layer weights, and wherein said minimization is inactivated when the sum of the weights is below a threshold value.

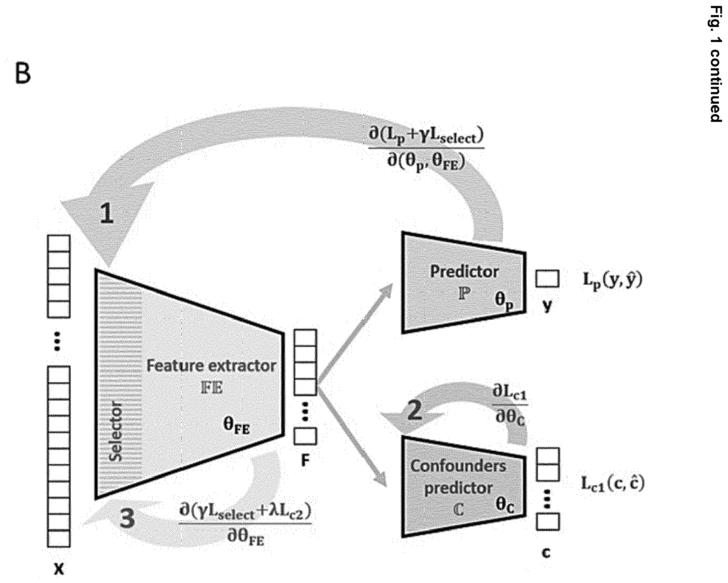

In preferred embodiments the neural net is trained in an adversarial fashion by preferably cyclically repeating the following steps at least once (see also Figure 1):

    i. The parameters of FE and P are optimized to minimize the prediction error of P.

    ii. The parameter of C are optimized to minimize the error of C.

    iii. The parameter of FE are optimized to maximize the error of C.

In embodiments of the method according to the present invention the feature extractor (FE) is responsible for reducing the input information to a vector $\vec{F}$ with a lower dimensionality, whose elements are a non-linear combination of the inputs, the predictor (P), uses the output of FE to predict the an age-related mortality or disease, such as time to death and/or the mortality risk and/or the risk of age-related disease, paired to the input data $\vec{x}$, and the confounder predictor (C), uses the output of FE to predict a vector of at least one categorical and/or continuous confounder(s), which are associated to the input data $\vec{x}$.

In embodiments of the method according to the present invention the neural network is trained by adversarial machine learning by cyclical repetition of the following steps, comprising at least one repetition:

    a) optimizing the parameters of FE and P to minimize the prediction error of P,

    b) optimizing the parameter of C to minimize the error of C,

c) optimizing the parameter of FE to maximize the error of C.

In embodiments of the method according to the present invention the adversarial learning framework, is a neural network comprising the element(s): a feature extractor (FE), and/or a predictor (P), and/or a confounder predictor (C).

In embodiments of the method according to the present invention the adversarial learning framework, is a neural network comprising three elements: a feature extractor (FE), a predictor (P), and a confounder predictor (C).

In preferred embodiments the present method uses a selector layer to filter/select only the most relevant inputs (input data) for prediction, thereby also achieving the effect of reducing the overfitting problem. Preferably the selector layer works by multiplying each input element by either 0 (and thus eliminating such element from further processing in the following layers) or 1, leaving the rest of network the possibility to use this datum. In order to choose which weight to assign to each element, during the training process, a compromise is searched between the optimization of the overall network performance in the specified task, and a constraint on the sum of the weights of the first layer that encourages the net to assign some zero weights to the inputs.

Another element of novelty of the present invention over the prior art is that in the implementation of the present method, the regularization used to encourage the assignment of zero weights is a cut-L1 norm. It consists in a standard L1 norm regularization that imposes the net to minimize the sum of the selector layer weights that is inactivated when the sum of the weights goes below a certain selectable threshold. This feature surprisingly facilitates to influence the number of input features that are desired by the user or are advantageous or expedient to be selected by the net. An issue with the use of binary weights is that during the training it is difficult to estimate how to optimize their value to minimize the prediction error, because binary weights are not differentiable. In one embodiment of the method, this problem is solved by a one-to-one layer that uses binary weights during the prediction phase and continuous weights for parameter optimization during training.

The use of one-to-one layers with binary weights has been adopted also by Trelin et al., 2019 and other works. However, the solution proposed by Trelin et al. is not suitable to solve the problem underlying the present invention, as Trelin et al. developed an inference based on probabilistic weights, which makes it impossible to univocally identify a list of relevant inputs.

In summary, in preferred embodiments, the present invention provides a novel architecture, such as depicted in Fig. 1, that is particularly suited for highly accurate and reliable age-related mortality or disease, e.g., time to death and/or the mortality risk and/or the risk of age-related disease, predictions, composed preferably by an initial selector that filters the input data followed by a tripartite structure trained in an adversarial fashion to avoid learning from confounders. This novel architecture with the aforementioned features trained on biological data obtained from, for example, multiple reference subjects, such as animal models or human subjects, paired with their time to death information and/or other phenotypic variables that are relevant for age-related diseases produces a biological timer, resilient to overfitting and confounders, from which a list of the most relevant input variables for prediction is easily accessible by reading the weights assigned to the first layer.

In embodiments the present invention provides a method for predicting the lifespan, an age-related mortality or disease, such as the time to death or the mortality risk and/or risk of age-related disease of a subject, wherein biological data, i.e., biochemical- and/or molecular- and/or genetic- and/or phenotypic data, of a subject, or any combination thereof are provided together with a list of variables related either to the individual, or to the sample or to the generation process of the biological/molecular/genetic/phenotypic/physiological data that the user wishes the prediction to be independent from. These variables may in embodiments be selected by the user and are called confounding variables. Based on this data the time to death for the subject or risk for aging-related diseases is predicted by analyzing the data with an algorithm, wherein the algorithm comprises a (deep) neural network, which is trained on at least one reference dataset comprising biological data , e.g., biochemical-, molecular, proteomic, genetic- or phenotypic-data or their combination, for a set of reference subjects for which the variable(s) to be predicted are known by preferably applying a selector layer to filter input data, and an adversarial learning framework, that removes from the input data the information related to the confounders and thus makes the time to death or the risk for aging-related diseases predictions independent from the confounder variables, that were preferably set by the user. In preferred embodiments there are applied a selector layer to select data variables, and an adversarial learning framework, comprising a feature extractor (FE), and/or a predictor (P), and/or a confounder predictor (C).

In embodiments of the method according to the present invention the biological data is selected from the group comprising genetic-, genomic-, proteomic-, metabolic-, immunological-, transcriptomic- or phenotypic-data or any combination thereof. In other words, the biological data may comprise data on genetic variations, DNA methylation, histone enzymatic modifications, RNA abundance, RNA splicing, RNA modifications, metabolites, protein abundance, protein modifications or re-localizations, protein interactions and/or phenotypic-data.

In embodiments of the method according to the present invention the biological data of the subject is selected from the group comprising genome data, epigenome data, transcriptome data, proteome data, metabolome data or interactome data.

In one embodiment the biological data is or comprises molecular data. In one embodiment the biological data is or comprises biochemical data. In one embodiment the biological data is or comprises medical or biomedical data. In one embodiment the biological data is or comprises phenotypic and/or clinical data.

In embodiments of the method according to the present invention the subject is a human, or an animal model, preferably a fish, a mouse or another vertebrate.

In some embodiments the reference subjects from which the training data is derived are a certain model organism or model animal with a relatively short lifespan, while the subject for which the lifespan is predicted is a human or higher animal.

In some embodiments the model is trained on reference individuals of a certain species. Then, the same or other individuals of the same species are imposed with an intervention, and the already trained model is used to evaluate the effect of the intervention through its predictions. In some of these embodiments, the species is a short lived killifish. In some of these embodiments, the intervention consists in the administration of pharmacological substances or dietary changes.

In some embodiments the reference subjects from which the training data is derived may be short lived killifish, then different individuals are imposed with an intervention (e.g. a pharmaceutical treatment), while the trained model according to the invention is then used to predict the effect of the intervention on the lifespan of a subject, based on the subject's biological data, wherein the subject is not the species used for training but a human or another relatively long-living model animal (at least compared to the reference model animal), such as a mouse or a rat. In such embodiments the knowledge gained from model animals with a short lifespan can be employed to predict the influence of a certain factor, e.g., a substance or other influence, on the lifespan and/or time to death and/or the mortality risk and/or the risk of age-related disease of a human or model animal with a longer lifespan, without the need to test the factor or influence directly on these parameters of the subject with a long lifespan, such as a human or an animal model. This approach offers multiple advantages and advantageous applications of the resent method e.g., for the testing the influence of substances, pharmaceuticals of stressors on the lifespan, e.g., of human subjects.

Herein "multiple reference subjects" preferably refers to more than one reference subject, even more preferably to more than 5, or even more than 10 reference subjects.

In embodiments the present method may also be used to test the influence of a drug on parameters other than lifespan, for example, to prioritize or selected drugs for clinical studies focused on human subjects or patients.

In embodiments of the method according to the present invention the at least one reference dataset is derived from multiple reference subjects, which has a shorter lifespan or life expectancy than the subject which lifespan and/or time to death and/or mortality risk and/or risk of age-related disease is predicted by the present method in step ii.

In embodiments of the method according to the present invention the at least one reference dataset is derived from multiple reference subjects, wherein a reference subject is an animal or model animal and wherein the subject which lifespan and/or time to death and/or mortality risk and/or risk of age-related disease is predicted by the present method in step ii. is a human. In some of said embodiments the model animal is selected from the group comprising fish, such as zebrafish (*Danio rerio*) or killifish (*Nothobranchius Furzeri*)), mice (*Mus musculus*), rats (*Rattus rattus* or *Rattus norvegicus*), or invertebrates, such as nematodes (*Caenorhabditis elegans*) or insects (*Drosophila melanogaster or Apis mellifera*).

In preferred embodiments the adversarial learning framework removes the information related to confounders from the input data and thus facilitates the time to death or the risk for aging-related diseases predictions to be independent from the confounder variables. It was surprising that the present implementation of the selector layer and its combination with the adversarial learning achieves the benefits described herein. None of the two methods has ever been applied to the development of a biological time to death and/or the mortality risk and/or the risk of age-related disease predictor or clock or to the analysis of "omics" data in general.

In addition, implementing the two strategies in a single net should not be considered a simple juxtaposition of elements, because their unifying implementation required some complicated and non-obvious technical decisions by the inventors, such as how to incorporate the regularization of the selector into the adversarial learning. Unexpectedly, the advantages of using the combination of these features are greater than those of using them separately (hence synergistic). For instance, by incorporating the regularization of the selector in both the rounds in which the FE is trained, the adversarial purpose is strengthened. In fact, the net will be encouraged to filter away those input data that heavily depend on the confounders and to retain those that minimize the error of P. This combined action also has the advantageous and surprising effect of reducing training time.

One of the advantages of the invention is that the present algorithm can be used on any kind of high-dimensional biological data (transcriptome, proteome, DNA sequence variation, imaging, etc.) and/or on any organism, i.e., plants, microorganisms, animals and humans.

In addition, thanks to the adversarial learning, the present method is able to extract patterns that are resilient to the type of tissue or sample analyzed, different experimental/environmental conditions, the sex of the subject, etc.

The special feature of preferred embodiments of the present invention is the use of a selector layer that makes it possible to avoid overfitting, and wherein the parametrized regularization allows influencing the number of input features to select from all those available.

All these properties make the present method a very versatile tool to discover new and diverse biomarkers of aging, age-associated diseases or any other physiological/pathological condition that can be assessed longitudinally and that can be used differently based on the application/research of interest. Most predictors of the prior art require a careful design of the training process to avoid overfitting and confounding effects. The present invention, however, thanks to the combined adoption of the adversarial learning and the selector layer, ensures the algorithm to be applicable, with reliable results, to almost any set of data (if the confounders are known and a reasonable number of inputs to select is set).

Confounding variables or confounders are related either to the individual, or to the sample and/or to the generation process of the biological data, e.g., molecular, genetic and/or phenotypic data, that a user, patient or physician wants the prediction to be independent from.

Hence, in embodiments of the method according to the present invention the at least one confounder(s) is selected from the group comprising individual features, sample properties and technical variables relevant for acquiring the data.

In one aspect the present invention relates to the use of the method according to the invention for predicting an age-related mortality or disease for the subject. In another aspect the present invention relates to the use of the method according to the invention for predicting the lifespan and/or time to death for the subject. In another aspect the present invention relates to the use of the method according to the invention for predicting the mortality risk and/or risk of age-related disease for the subject.

In embodiments the present method can be used to reliably predict individual lifespan or any other age-related condition of a subject, wherein the input data or dataset preferably comprises data of a large number of reference subjects, preferably human subjects, and was acquired from an heterogenous cohort of subjects annotated with the information regarding all the possible sources of variations.

In another aspect the present invention relates to the use of the method according to the invention for predicting the influence of a substance on the lifespan of a subject, wherein biological data of the subject are obtained at least after receiving the treatment.

In another aspect the present invention relates to the use of the method according to the invention for predicting the influence of a stressor or external influence or factor on the lifespan of a subject, wherein biological data of the subject are obtained at least after being exposed to the stressor or external influence or factor. Such stressor or external influence or factor may be physical or psychological stress, physical activity, nutrient deprivation, nutritional or food overabundancy or oversupply, starvation, fear or anxiety, exposure to pathogens or sleep deprivation.

In general, the present method may be used to examine the influence of any factor, or even any combination of factors, on the lifespan, and age-related mortality or disease, the time to death, the mortality risk and/or the susceptibility to age-related diseases of an individual. In embodiments the individuals would be exposed to these factor(s) or substances and reference samples would be taken and analyzed according to the present invention. The obtained data will be compared with a previously generated reference/training data, such that the influence of said factors could be extrapolated or translated by the algorithm of the present invention to the lifespan, and/or age-related mortality or disease, and/or time to death and/or the mortality risk and/or the risk of age-related disease of a single an individual, whose sample is analyzed.

In other embodiments, subject(s) would be exposed to these factor(s) or substances and reference samples would be taken before and after exposure and analyzed according to the present invention and the algorithm will detect a change of the lifespan, age-related mortality or disease, or time to death of a single individual induced by exposure to said factors.

In other embodiments the present method can be used for the design of individual screening programs. For instance, if now healthcare guidelines indicate a one-fits-all age for screening for breast or prostate cancer, the present method could be used to reliably identify subjects with an accelerated aging process and suggest them to undergo further health screenings or anticipate screenings.

The present method may be further used to design biomarkers of aging or age-associated conditions and for the development of diagnostic and/or medical devices based on them.

In addition, in embodiments the present method may be used in experimental paradigms such as the one described in Example 1 to advance the discovery of new drugs, treatments, e.g., substances, and to accelerate clinical trials.

In one aspect, the invention relates to a software or computer program product for predicting the lifespan of a subject, wherein when said software or computer program product is executed, the following steps are conducted:

i. receiving biological data of the subject,

ii. receiving a list of confounding variables,

iii. predicting an age-related mortality or disease for the subject by analyzing the data with an algorithm,

wherein the algorithm comprises a deep neural network, which is trained on at least one reference dataset comprising biological data of multiple reference subjects by applying:

a) a selector layer to select data variables, and

b) an adversarial learning framework, that removes from the input data the information related to the confounding variables, and

transmitting and optionally displaying an output of the software or computer program product to a graphical user interface.

In one embodiment predicting an age-related mortality or disease comprises predicting the time to death and/or the mortality risk and/or the risk of age-related disease for the subject.

In one embodiment of the software or computer program product according to the invention, predicting the lifespan of a subject comprises predicting the healthspan of a subject, either instead of, or in addition to predicting the lifespan of a subject, wherein the healthspan is the timespan in which the subject is free from chronic disease, (invalidating) disease, disabilities of aging and/or age-related disease.

In one embodiment of the software or computer program product according to the invention, the adversarial learning framework, is a neural network comprising three elements

I. a feature extractor (FE),

II. a predictor (P), and

III. a confounder predictor (C).

Particular aspects of the present invention may be computer-implemented. Accordingly in embodiments the present method may be a computer-implemented method. The person skilled in the art is aware of which aspects and features of the present invention may be computer-implemented.

In a further aspect the present invention relates to a computer-readable storage device, comprising a software or computer program product according to the invention.

In a further aspect the present invention relates to a computer-readable storage medium having stored thereon the software or computer program product according to the invention.

Example 2 shows that an embodiment of the present method accurately predicts time to death for individuals of the *N. furzeri* fish. Another embodiment, also described in Example 2, predicts a higher age for individuals belonging to a strain with shorter lifespan. These results indicate that the present invention provides a standardized technology platform for testing pharmacological interventions with lifespan extension potentially using a model animal, such as the fish *N. furzeri* (Killifish), by applying the proprietary lifespan predictor according to the present invention (in this experiment as a "Killifish Intervention Platform" (KIP)). The present method provides information of potential human benefit in less time and at a fraction of the cost of the current gold standard methods, which is lifelong treatment of mice.

From the current point of view, the present invention has the following further options for practical application: The present computer implemented method may also be applied as a platform to test the lifeprolonging effect of pharmacological substances, or to develop new biomarkers of aging or longevity, or even for "age estimation" based on the use of biological data and/or biomarkers of an individual.

Each feature of the invention that is disclosed in the context of one aspect of the invention is herewith also disclosed in the context of the other inventive aspects disclosed herein. Accordingly, embodiments and features of the invention described with respect to the method disclosed herein, are considered to be disclosed with respect to each and every other aspect of the disclosure, such that features characterizing one embodiment of the present method, may be employed to characterize another embodiment of the method or it's use, the computer program product or device and vice-versa. The various aspects of the invention are unified by, benefit from, are based on and/or are linked by the common and surprising finding of the unexpected advantageous effects of the present method to reliably predict the lifespan or time to death and/or the mortality risk and/or the risk of age-related disease of an individual, based on biological data of the individual, by employing a neural net that was trained using the combination of a selector layer and an adversarial learning framework to learn from biological reference data.

**DETAILED DESCRIPTION OF THE INVENTION**

The present invention relates in preferred embodiments to a method for predicting the lifespan or age-related mortality or disease, such as the time to death and/or the mortality risk and/or the risk of age-related disease, of an individual by applying an algorithm comprising a neural network that comprises and takes advantage of a novel architecture, such as depicted in Fig. 1. Hence, this preferred method is particularly suited for highly accurate and reliable time to death and/or mortality risk and/or risk of age-related disease predictions. The method is composed preferably by an initial selector that filters the input data followed by a tripartite structure trained in an adversarial fashion to avoid learning from confounders. This novel architecture with the features, described herein in detail, if trained on biological data obtained from, for example, multiple reference subjects, such as animal models or human subjects paired with their lifespan information, becomes a precise timer, resilient to overfitting and confounders, from which a list of the most relevant input variables for prediction is easily accessible by reading the weights assigned to the first layer.

In the context of the present invention the term "lifespan" may herein refer to the length of time (e.g., number of years/months/days) for which a subject, person or animal lives. In some embodiments the term "lifespan" may also refer to the "life expectancy" of a subject, which can be defined as the total number of years that a subject, e.g., animal or human, is expected or prognosed to live (from birth to death). In some embodiments the term "lifespan" may refer to the "time to death" of a subject, which may be calculated in years or months from the date of taking a biological sample and/or collecting physiological data of the subject or starting from its date of birth.

In embodiments the "lifespan" of a subject can be predicted based on the mortality risk and/or the risk of age-related diseases of a subject. In embodiments the term "lifespan" may comprise or refer to an age-related mortality or age-related disease. In embodiments "lifespan" may comprise or refer to the time to death, and/or the mortality risk and/or the risk of age-related disease of a subject. In some embodiments the age-related mortality or disease, and/or the risk of mortality and/or the age-related diseases influence, are directly or indirectly related to, are linked to, and/or are relevant for the (predicted) lifespan and/or mortality and/or time to death of a subject.

In embodiments the term "lifespan" may comprise or refer to the "healthspan" of a subject. Herein healthspan preferably refers to the time the subject/individual is free of (invalidating or chronic) diseases and/or age- or disease-related disabilities, in other words, healthspan may herein refer to the period of a subject's life in which it is in good health and free from chronic disease, invalidating disease, disabilities of aging (or age-related disabilities) and/or age-related disease. For example, in some embodiments of the present invention the neural network will predict the "healthspan" of a subject, either in addition to predicting the lifespan or instead of. In embodiments the "healthspan" of a subject can be predicted based on the mortality risk and/or the risk of age-related diseases of a subject. The healthspan of a subject is preferably calculated in years or months either from the date of taking a biological sample and/or collecting physiological data of the subject, or starting from its date of birth.

The term "mortality" in general refers to the state of being mortal (destined to die). In medicine and in embodiments herein, the term may also be used for the life expectancy, the chance of death or risk of death of a subject within a certain period of time. Mortality may in embodiments be prognosed, calculated or determined for subjects having or being suspected of having a certain disease, who comprise a certain genetic background or genetic variants and/or based on certain determined clinical, biological and/or molecular values, markers or parameters of a subject, e.g., as described herein.

Herein, "age-related disease" may refer to any disease occurring in elderly patients, such as patients aged over 30, 40, preferably over 50, more preferably over 60 years of age. Herein age-related diseases comprise, without being limited to, diseases such as Alzheimer's disease, dementia, cancer, leukemia, type 2 diabetes, arthritis, rheumatic disease, neurodegenerative diseases, atherosclerosis, cardiovascular disease, cataracts, osteoporosis, hypertension, frailty and sarcopenia, chronic obstructive pulmonary disease (COPD), and Parkinson disease. In embodiments of the present invention an "age-related disease" may comprise and/or refer to a risk of age-related disease of the subject.

Herein the term "age-related mortality" preferably relates to the mortality of a subject that is related to, caused by, associated with and/or is dependent on the age, preferably a higher age, e.g., 30, 40, 50, 60, 70, 80, 90 or even 100 or more years of age, of a subject, and that is preferably independent of external causes of mortality, death and/or disease, such as accidents, violence inflicted to a subject from outside, and/or other external causes that are independent of or unrelated to the health, genetics and/or age of a subject. In embodiments of the present invention an "age-related mortality" may comprise and/or refer to the time to death and/or a mortality risk of the subject.

Herein the "risk of mortality" refers to the likelihood and/or chance of death of a subject. Preferably, the risk of mortality refers to a likelihood of death of a subject within any given time frame or period of time. For example, from the time point of assessment a predicted mortality risk can be assigned for a future time period, using the methods disclosed herein. In preferred embodiments the risk of mortality is independent of external causes of mortality, death and/or disease, which are independent of or unrelated to the health, genetics and/or age of a subject, such as accidents or violence inflicted to a subject from outside.

In the context of the present invention the term "subject" refers to an individual, a patient, a human, an animal, a model animal, a mammal, a vertebrate, preferably a model animal or a human. In preferred embodiments the subject is a human, or an animal model, such as preferably a fish (e.g. zebrafish (*Danio rerio*) or killifish (*Nothobranchius Furzeri*)), a mouse (*Mus musculus*), a rat (*Rattus rattus* or *Rattus norvegicus*) or any other suitable vertebrate. The subject may further be an invertebrate, a nematode or an insect, such as, e.g., *Drosophila melanogaster, Apis mellifera* or *Caenorhabditis elegans.* Herein the terms "subject", "patient" and "individual" may be used interchangeably.

Herein a "sample" may be taken from a subject, a patient, a cell culture of patient cells or cell lines, an animal, or a cell culture of animal cells or cell lines of a biopsy, a blood sample, a tissue sample, or an environmental sample. Basically, any kind of sample that is suspected to contain biological information of interest. As used herein, the term "sample" is a biological sample that is obtained or isolated from the subject, Sample as used herein may, e.g., refer to a sample of bodily fluid, tissue or surface (e.g., mucosal swap sample) obtained for the purpose of diagnosis, prognosis, or evaluation of a subject of interest. In case of a liquid sample, or a liquid biopsy the sample may be in embodiments a sample of a bodily fluid, such as blood, serum, plasma, cerebrospinal fluid, urine, saliva, sputum, pleural effusions, a cellular extract, and the like. In further embodiment the sample may be a solid sample, such as a biopsy, a tissue sample, a cell culture sample, cells, a tissue sample, a tissue biopsy, a stool sample or a swap-derived sample.

In the context of the present invention the term "biological input data" or "biological data" or "input data" or simply "input", may refer to any representation of data from a biological source, basically any suitable biological, biochemical and/or molecular data that can be processed by the inventive method. By way of example, molecular or biochemical data may comprise genome or genomics data, epigenome or epigenomics data, transcriptome or transcriptomics data, proteome or proteomics data, metabolome or metabolomics data, interactome or interactomics data, medical, clinical or molecular imaging data, and/or physiological data. The data may further comprise or consist of microbiological data or chemical compounds detected in a sample, e.g., bacteria strains or hormones or vitamins in a sample of a subject. In preferred embodiments the biological data of the subject is selected from the group comprising genome data, epigenome data, transcriptome data, proteome data, metabolome data or interactome data, data on genetic variations, DNA methylation, histone enzymatic modifications, RNA abundance, RNA splicing, RNA modifications, metabolites, protein abundance, protein modifications or re-localizations, protein interaction, cytotoxicity data, clinical and cellular imaging data and/or phenotypic data. By way of example the input data may be generated or acquired using one of the methods selected from the group comprising nucleic acid sequencing (e.g., next generation sequencing, NGS), mass spectrometry (MS), Western Blot, cytotoxicity screening, FACS (Fluorescence Activated Cell Sorting) analysis, digital- or real-time PCR, microarray, cytokine-arrays, Nanostring, microscopy, immunofluorescence, immunostaining, clinical examination devices (e.g., X-ray or MRI/MRT) or any other biochemical analysis of a sample (e.g., chemical or microbiological analysis of a blood, urine or feces sample) and/or medical or physiological examination.

Herein "phenotypic data" may comprise any kind of clinical or biometrical or functional information regarding a subject or patient, such as blood chemistry, arterial pressure, measures of cardiovascular function, BMI, diagnosed diseases and/or symptoms, byway of example coronary artery/heart disease, kidney disease, heart failure, hypertension, (cardio)vascular disease, cancer, neurodegenerative diseases, diabetes, etc., relevant habits, diet, relevant demographic data, comprising age, ethnicity, sex, or even socioeconomic data, such as place of residence and/or birth, or educational status.

Herein a "reference dataset" comprises data of preferably more than one or multiple reference subjects, even more preferably of more than 10 reference subjects. Preferably the reference data and/or reference dataset comprises the same kind of (biological) data, as the input data, e.g., proteomics data, and was preferably acquired using the same or a comparable or equivalent method, e.g., mass spectrometry.

The term "feature" refers in the context of machine learning and pattern recognition, to an individual measurable property or characteristic of a phenomenon. Choosing informative features is a crucial element of effective algorithms in pattern recognition, classification and regression.

The term "machine learning (ML)" refers to a field of inquiry devoted to understanding and building methods that 'learn', that is, methods that leverage data to improve performance on some set of tasks. It is seen as a part of artificial intelligence. Machine learning algorithms build a model based on sample data, known as training data, in order to make predictions or decisions without being explicitly programmed to do so.

A "neural network" refers herein in the context of machine learning to a specific type of machine learning in which the input is transformed through a network of artificial neurons or nodes, organized in a sequence of layers. The connections of the biological neuron are preferably modeled in artificial neural networks as weights between nodes. All the signals received by a neuron are preferably combined and further processed by an activation function.

Herein "deep learning" is part of a subgroup of machine learning methods based on artificial neural networks that are said to be "deep" (deep neural networks), meaning that they are composed by multiple layers (broadly more than 3) through which the data is transformed. Deeplearning architectures called "deep neural networks (DNN)s" have been applied to fields including bioinformatics, where they can produce results comparable to and in some cases surpassing human expert performance.

Adversarial machine learning describes a technique to train machine learning models to optimize a cost function that is based on maximizing the capability of the model of predicting the target, while at the same time minimizing some other constraint (like the capability of predicting confounders, in the case at hand).

In preferred embodiments the DNN is forced in the "adversarial learning framework" or setup to select a set of features that maximizes the performance prediction of the desired task, but at the same time minimizes the performance prediction of the possible confounders. Besides preventing learning from possible confounders, this sort of 'tug of war' indirectly reduces overfitting, because it forces the algorithm to find alternative patterns with respect to the one that would simply maximize the performances alone.

In embodiments herein the "feature extractor" (FE) is a part of the neural net or DNN that processes the input to extract features ($F_i$). It starts with a one-to-one binary layer that acts as a binary feature "selector", by multiplying each of its inputs by either zero or one.

The pattern learned by the DNN is notoriously very difficult to extract and analyze. Therefore, preferably a binary feature selector is put as a first layer of the DNN, that assigns a binary weight (0 or 1) to each input data point. This allows to filter the genes of interest to predict the output. Preferably the selector is also regularized in order to partially control the number of data points to select. Thanks to the presence of the binary selector, it is possible to extract the list of data points chosen by the algorithm to predict the output. Preferably, as already mentioned for the EN, the research of a sparse pattern contributes to avoid overfitting.

Choosing the value of these weights during back-propagation is a difficult task, because their integer nature does not allow to calculate the gradient to be minimized during the training process. For this reason, the present method uses continuous weights constrained in the range [0, 1] for the calculation of the gradient, but rounds them to the nearest integer during the calculation of the prediction (i.e., the inference phase). To force this filter layer or "selector layer" to sparsely select the features, a local *L1* regularization is introduced. However, the inventors empirically noticed that in this setup, the *L1* regularization pushes too many weights below 0.5 and, given that the gradient calculation does not see the rounding operation, it is difficult to increase their value afterwards. This phenomenon occurs especially in long training sessions. To solve this issue, the inventors set a cut-off threshold t on the sum of the absolute values of the weights $w_k$ below which *L1* regularization is no longer applied. In conclusion, in embodiments this "selector layer", with respect to the rest of the FE, presents the following additional penalization:

$$L_{selec} = \max\left(\left(\sum_k |w_k|\right) - t, 0\right)$$

Thus, herein the term "cut-L1 norm regularization" refers to a standard L1 norm regularization that imposes the net to minimize the sum of the selector layer weights that is inactivated when the sum of the weights goes below a certain selectable threshold.

Apart from the selector layer, in embodiments the rest of the FE is composed by fully connected layers occasionally interspersed by dropout ones.

In embodiments another part of the neural net is the "predictor" P that takes as input the features ($F_i$), generated by the FE, and tries to predict the target output ($y_i$), producing an estimated $\hat{y}_i$. The structure of P is composed by fully connected layers and its parameters are indicated with $\theta_P$.

In embodiments another part of the neural net is the "confounders predictor" ($C$) that, similarly to the predictor ($P$), is composed by fully connected layers and that tries to predict the confounders ($c_i$) from the features ($F_i$). Its parameters are indicated with $\theta_c$. The present method may be employed in different embodiments using different layer setups for each of its different components (e.g., in one embodiment the FE, the C and the P) within the adversarial learning framework. In different embodiments these layers can differ for the type of mathematical operation performed by each neuron, by the number of neurons and/or by the number and organization of the neuronal connections. The use of a diverse set of layers does not affect the purpose of the method, i.e., making explainable lifespan/time-to-death/mortality/aging-related-risk predictions independent from a list of confounders using a set of input biological data, for example, as long as the method comprises certain features, such as the adoption of an adversarial learning framework and/or the use of a binary selector layer as first layer processing the input data, wherein the method preferably comprises both the adversarial learning framework and the selector layer.

In preferred embodiments the herein described DNN architecture is used to train a "transcriptomic clock (TC)" and/or a "transcriptomic timer (TT)" on biological data. Preferably the development of the TC and TT can be formally described as a regression problem for which each of the *N* instances i is a triplet, composed by: a vector of input features, its target output and a vector of confounders (containing both categorical and continuous variables): $\{(X_i, y_i, c_i)\}_{i=1}^{N}$ ,

wherein the aim is to predict $y_i$ from a set of features $F_i$ derived from a subset of the input $X_i$ while avoiding overfitting and ensuring that $F_i$ does not contain information about the confounders $c_i$.

The statistical term "overfitting" in general refers to the concept of data analyses corresponding too closely or exactly to a particular (training) dataset. Computational models suffering from overfitting in general lack transferability to other datasets, the ability to fit to other datasets, additional data or are unable to reliably predict future findings. An overfitted model is a mathematical model that has over-specialized its behavior to training data. The inherent nature of overfitting is the unnoticed extraction of some of the residual variation (i.e., the noise) as if that variation represented underlying data structure.

Herein the term "graphical user interface" or "GUI" relates to a form of user interface that allows a user to interact with electronic devices through a graphical representation, graphical icons and/or audio-visual or haptic instead of text-based user interfaces, typed command labels or text navigation. Herein a GUI may be a system of interactive visual components for computer software that displays objects, instructions or data that convey information and represent actions that can be taken by as user.

In the context of the present invention the term "adjusting" comprises the meaning of adjusting, fitting, changing, amending, modifying or adapting. For example, when a parameter or weight is said to be "adjusted" during training it means that its value is modified in order to accomplish a specific task; in the machine learning context the task consists in minimizing/maximizing a certain cost function.

In statistics in general the process of weighting, or of assigning "weights", involves emphasizing the contribution of particular aspects of a phenomenon (or of a set of data) over others to an outcome or result; thereby highlighting those aspects in comparison to others in the analysis.

Accordingly, rather than each variable in the data set contributing equally to the final result, some of the data is adjusted to make a greater contribution than others.

In general, in the field of statistics, a "confounder" (also "confounding variable") is a variable that influences both the dependent variable and independent variable, causing a spurious association.

In the Machine Learning context, the dependent variable is the desired output (the lifespan, risk-of-mortality or aging-associated-risk) while each input data constitutes an independent variable (each gene, methylation site, et.).

Confounders can be characteristics of a subject or external factors. Confounding variables may be categorized according to their, for example, measurement instrument (measurement confounders; e.g. the biological data analyzed have been measured with different instruments), to the extraction and storage adopted to obtain the samples (pre-processing confounders: e.g. before measurement the samples could have been extracted using different techniques or stored in different conditions), to the post-processing operations (post-processing confounders: e.g. the measured data could have been normalized or processed in different ways), to sample characteristics (sample-related confounders: e.g. the tissue type, the cell type or the exact location of the sample in the tissue it was taken out), to individual characteristics (individualrelated confounders: e.g. the sex, age, weight, height, lifestyle of the individual or also past event in its life and the continuous or discontinuous use of drugs).

The term "at least one" may herein refer to at least one, more than one, at least two at least three, at least four, at least five, at least six, at least seven, at least eight, at least nine, at least ten, at least fifteen, at least twenty, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 500, 1000, 10.000.

**FIGURES**

The invention is further described by the following figures. These are not intended to limit the scope of the invention but represent preferred embodiments of aspects of the invention provided for greater illustration of the invention described herein.

**Figure 1:** Architecture of one of the embodiments of the deep neural network (DNN) algorithm employed by the method according to the invention. **(A)** General depiction of the architecture of a neural network according to preferred embodiments of the invention. **(B)** Depiction of an embodiment of the training process of the general architecture shown in (A). In embodiments the DNN requires a training set of $N$ instances, each of them i is a triplet, composed by: a vector of input features $X_i$, its target output $y_i$ and a vector of confounders $c_i$ (that may contain both categorical and continuous variables): $\{(X_i, y_i, c_i)\}_{i=1}^{N}$. The objective of the DNN is to adjust its weights ($\theta_{FE}, \theta_C$ and $\theta_P$) in order that each $X_i$ fed into this DNN is preliminary filtered by the selector, in order to avoid overfitting, then it is further reduced by the FE to a set of features $F_i$ *that* does not contain information about the confounders $c_i$, (meaning that the information contained in $F_i$ should maximize the prediction error of C) and from which it is possible to reliably

return its paired $y_i$, through P. Note that in the Figure, the symbol $\theta_{FE}$ is used to indicate all the parameters of the FE, including the selector's ones.

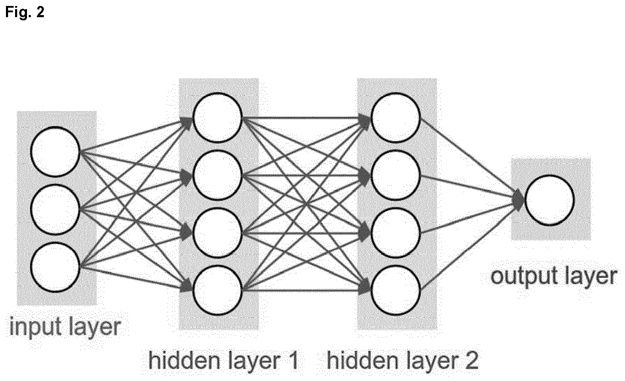

**Figure 2:** Example of a neural net composed solely by fully connected layers. As the image shows, fully connected layers connect all the inputs from one layer to each unit of the next layer. This operation is the most generic one and approximates any other mathematical operation. Apart from the selector layer, in embodiments the FE, C and P are composed by fully connected layers. In some embodiments FE is occasionally interspersed with dropout layers.

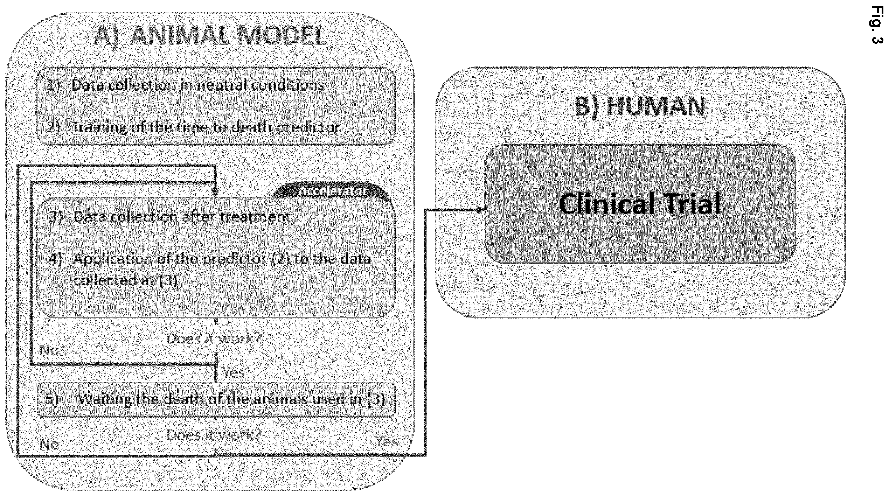

**Figure 3:** Schematic representation of one example of execution of an embodiment of the present method described in Example 1, namely a system for drug discovery applying the present method for predicting the time to death.

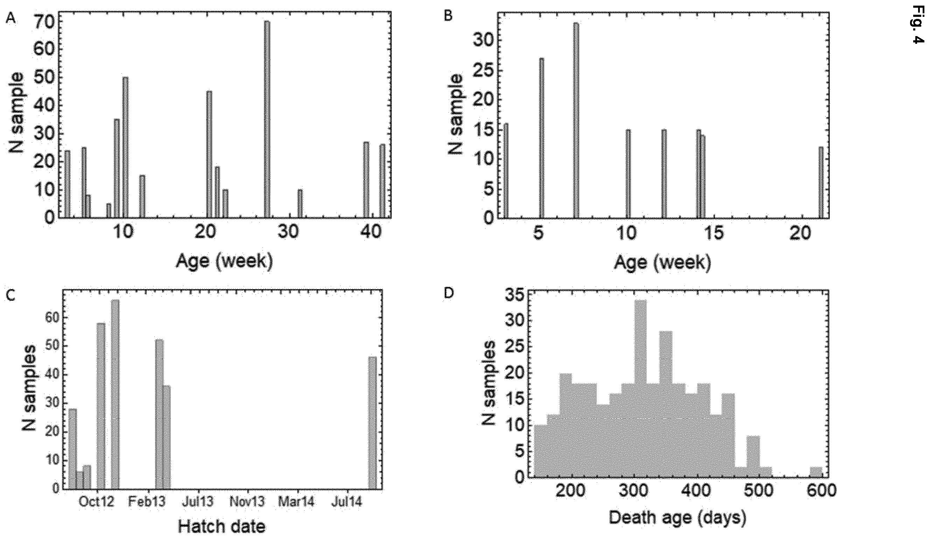

**Figure 4:** The Figure depicts demographic information of the datasets used in Example 2. **(A)** Age distribution of the samples belonging to dataset I, used to develop and test a Transcriptomic Clock (TC). **(B)** Age distribution of the samples belonging to dataset II, that was used to test the TC on a different strain with respect to the ones used to train it. **(C)** Hatch date distributions of the dataset III, used to develop and test the Transcriptomic Timer (TT). **(D)** Distribution of the age of death in dataset III, used to develop and test the TT.

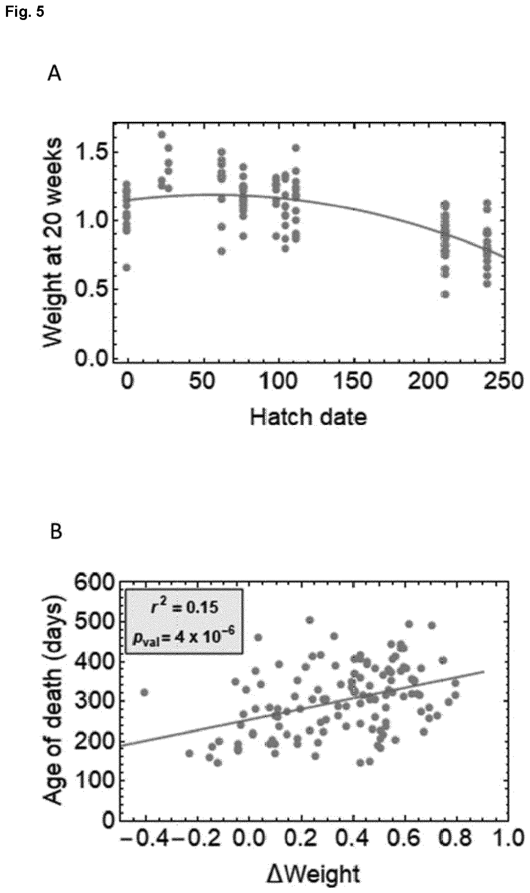

**Figure 5:** The Figure shows information about the datasets III that is relevant to plan the TT development, described in Example 2. **(A)** This Figure depicts the fishes' weight with respect to their hatch date. Note that the hatch dates are represented as the number of days passed by the first hatch date. As it can be observed hatch date and weight distributions are not independent. **(B)** This Figure depicts how the age of death depends on the weight variation between the 10th and the 20th week, which constitutes obviously an important biomarker of a fish's rate of growth, and consequently also of its aging speed.

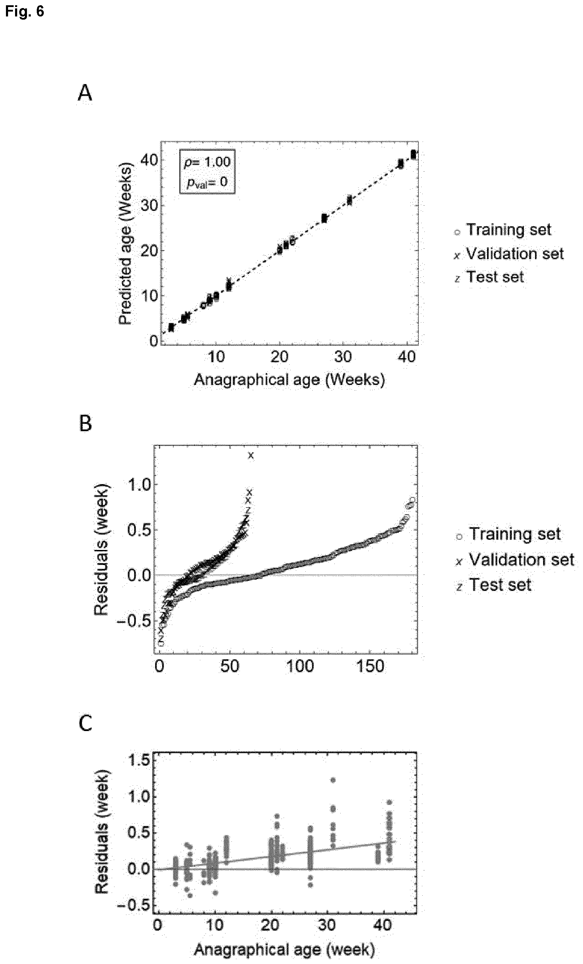

**Figure 6:** The Figure depicts results of the TC, developed in Example 2. **(A)** The figure depicts the correlation between the age predicted by TC and the anagraphical age. As it can be noted, the predictions on the training, validation and test sets are almost perfect, with no difference in the error distribution across the three of them. The figures (B) and (C) depict the TC residuals.

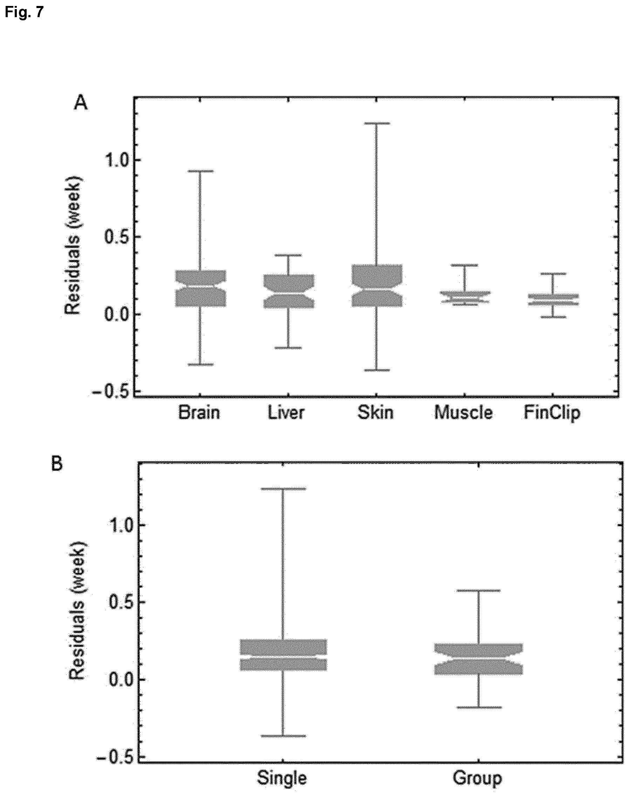

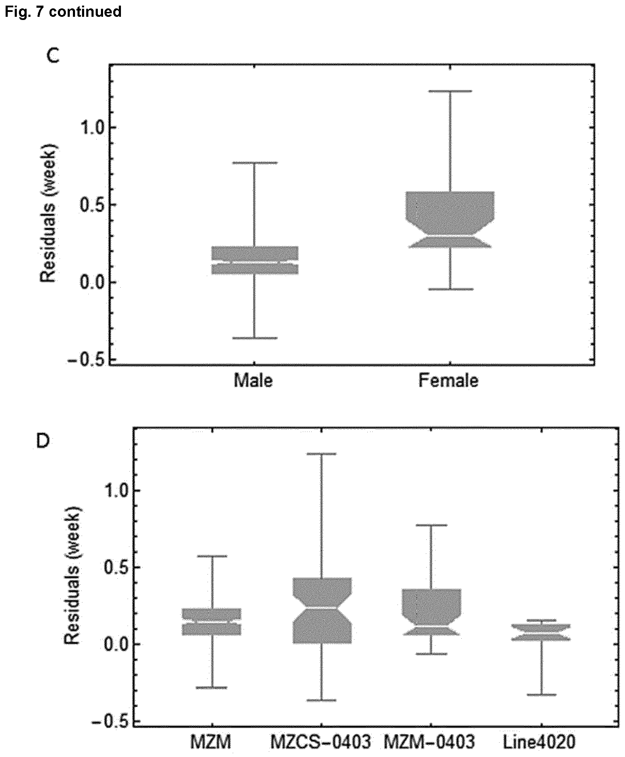

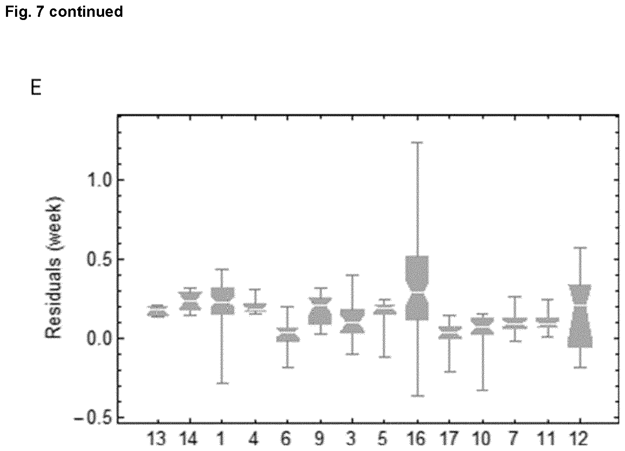

**Figure 7:** The Figure depicts the residuals of the TC (generated in Example 2) across different possibly confounding categories: **(A)** tissue, **(B)** housing, **(C)** sex, **(D)** strain and **(E)** batch. Taking into consideration the small entity of the residuals shown in the various plots it can be said that the developed TC shows no appreciable bias towards the reported categories.

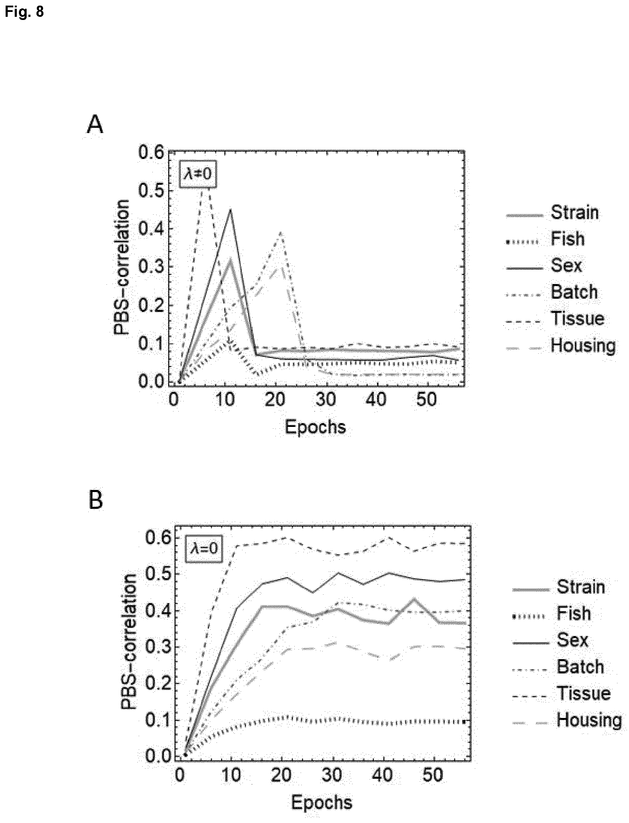

**Figure 8:** The Figure depicts the effects of the adversarial framework on the learning process of the TC, developed in Example 2. **(A)** The figure shows the variation across different epochs of the correlations between the predictions of C and the true values of different confounders when the adversarial framework is switched off ($\lambda = 0$). **(B)** The figure depicts the same correlations shown in (A), when the adversarial framework is switched on ($\lambda \neq 0$). As it can be seen without adversarial learning the model would have learned a pattern based on sex, housing and strain, which are in fact the most biased confounders in our dataset. While this framework allows to avoid learning from these variables.

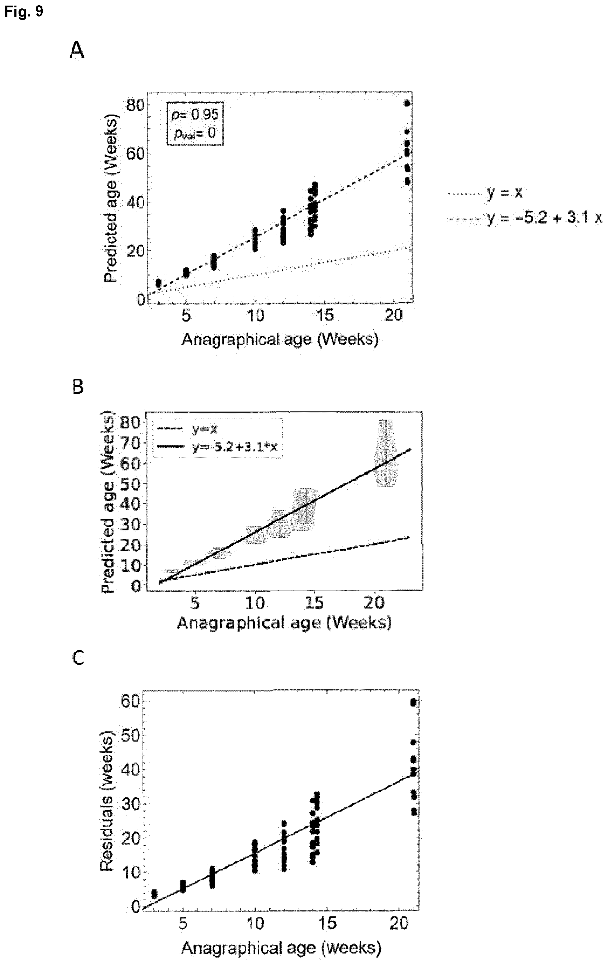

**Figure 9:** The Figure depicts the results of the application of the TC (developed in Example 2) on dataset II, which contains samples collected from a strain (GRZ) with a lower lifespan with respect to that of the ones used in training. The figures **(A)** and **(B)** depict the predictions on the GRZ samples using the TC. (A) shows the results in a dot-plot representation, while (B) depicts the data in a violin plot visualization. Figure **(C)** depicts the TC residuals on the GRZ samples fitted by a linear regression. As, expected the TC commits larger errors with respect to those shown in Fig 6 and systematically overestimates the age of GRZ samples. This suggests that the GRZ strain has a faster aging process characterized by a different transcriptomic pattern, which explains the larger errors committed.

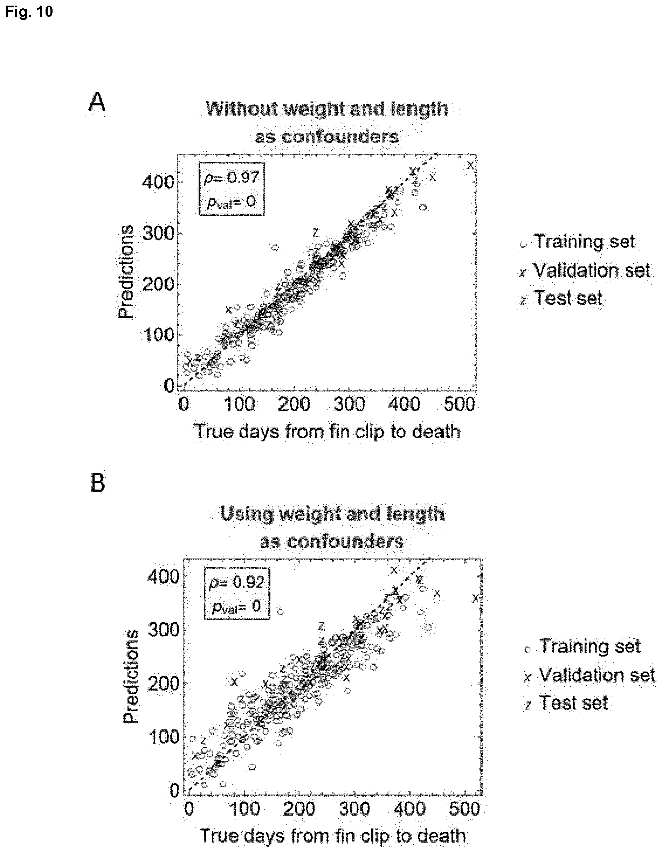

**Figure 10:** The Figure depicts the results of TT (developed in Example 2). The Figure shows the correlation between the true and predicted number of remaining days of life from sample acquisition, when the TT was trained with **(B)** and without **(A)** fish weight and length information as confounders.

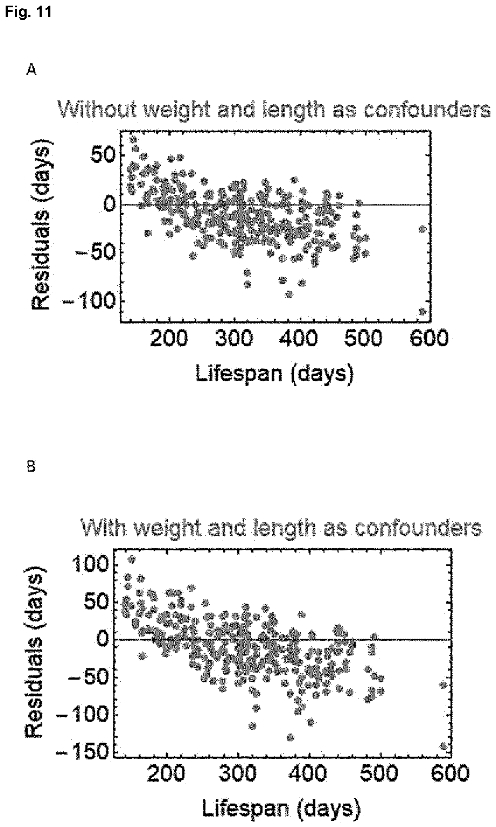

**Figure 11:** The Figure depicts the residuals of the TT (developed in Example 2) with respect to the entire lifespan. The graphs **(A)** and **(B)** show the residuals of the TT trained without and with fish weight and length information as confounders, respectively. The Figure shows that for both the TT versions, the number of remaining days of life are overestimated for short-living fishes and underestimated for long-living ones. The overestimation maybe due to the fact that fishes with a very short lifespan, probably did not die exclusively because of an innate faster aging process, but with some external contributing factors, that may have occurred after sample acquisition. The underestimation instead may be tied to non-linear aging progression at higher ages.

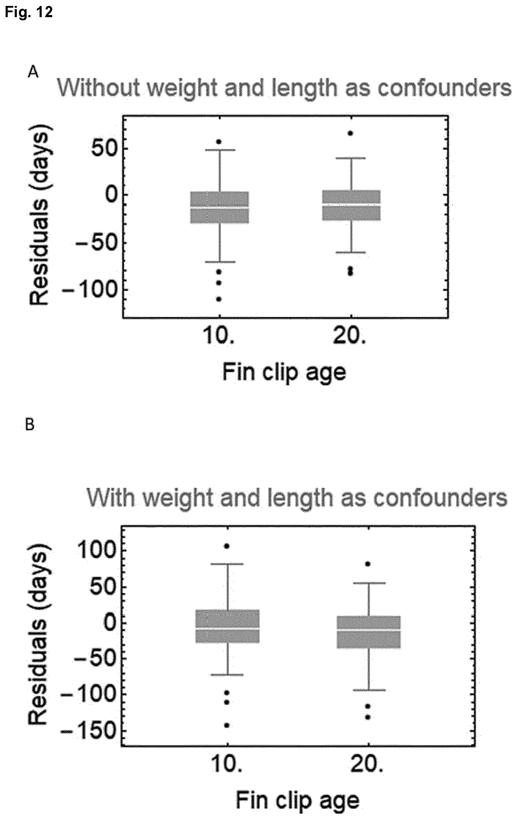

**Figure 12:** The Figure depicts the residuals of the TT (developed in Example 2) computed separately for two sampling ages (10 weeks and 20 weeks) collected in dataset III. The graphs of **(A)** and **(B)** show the residuals of the TT trained without and with fish weight and length information as confounders, respectively. The Figure shows that the prediction error does not depend on whether the data have been taken at 10 or 20 weeks of life of the fish.

## EXAMPLES

The invention is further described by the following examples. These are not intended to limit the scope of the invention but represent preferred embodiments of aspects of the invention provided for greater illustration of the invention described herein.

### Example 1

One example for the various applications of the present invention is its adoption to accelerate the discovery of new treatments/interventions able to improve health in aged population and/or prevent age-related diseases and/or reverse the effects of aging and/or stop or slow down the aging process.
One system for drug discovery based on the present method is depicted in Fig. 3.
Initially, a predictor of time to death for natural causes is trained based on data from an animal model collected in the absence of any intervention. Then, some new data are acquired after imposing the intervention to study (e.g., a specific drug) and the predictor previously developed according to the present invention is applied to these data to assess whether the time to death is delayed. This stage is called "accelerator" because, thanks to the use of the present method, that provides a surrogate measure of lifespan, it is not necessary to wait the death of the animals to decide whether the treatment is of interest or not. This provides a very significant cost reduction as it reduces the time of the experiment. This process is of particular interest when, for example, one lead compound needs to be identified among a number of candidates.
After the accelerator stage, it is possible to decide whether testing a new intervention or proceed with an experimental confirmation that the treated animals have an increased lifespan. If the experiment on the animal model has positive results it is possible to proceed with clinical trials on humans, as shown in Fig. 3, or it is possible to repeat the module A in another animal model, evolutionary closer to humans, before going to module B.

### Example 2

In the present Example the inventors used the present method, i.e. the DNN architecture described in Figure 1, to train a transcriptomic clock (TC) and a transcriptomic timer (TT) (i.e. an algorithm able to predict life expectancy from the transcriptome levels analyzed at only one point in life) on the gene expression data of the shortest living vertebrate *Nothobranchius Furzeri* (NF). The workflow can be articulated in three analyses:

1. Development and performance assessment of a pan-tissue and multi-strain TC.

2. Evaluation of the age prediction capabilities of the previous TC when applied to data belonging to a new, unseen, shorter-living strain.

3. Development and performance assessment of a single-sample TT.

In this embodiment the DNN architecture allows to address many problems in the prior art regarding the development of aging clocks and timers. First, the animal model used in the present Example has a short enough lifespan to collect biological samples paired with annotated lifestyle information and time of death. Second, the implementation of the adversarial learning framework allows to make the predictions independent from biological and technical confounders that may mislead the learning process. Third, the implementation of a selector layer allows to filter out input data in a data-driven fashion, while many previous works had to implement manual filtering processing, risking eliminating useful data while retaining 'useless' data. Fourth, the selector layer also allows to reduce the possibility of overfitting and of identifying a limited number of input data relevant for the aging process. Finally, the implementation of the constraint of the selector layer allows to actively influence the number of the selected input features.

The development of the TC and TT can be formally described as a regression problem for which each of the *N* instances i is a triplet, composed by: a vector of input features, its target output and a vector of confounders (containing both categorical and continuous variables): $\{(\mathbf{X}_i, y_i, \mathbf{c}_i)\}_{i=1}^{N}$

The inventors aimed to predict $y_i$ from a set of features $\boldsymbol{F}_i$ derived from a subset of the input $\boldsymbol{X}_i$ while avoiding overfitting and ensuring that $\boldsymbol{F}_i$ does not contain information about the confounders $c_i$. The inventors achieved this objective using the DNN illustrated in Fig. 1. The net is composed basically by 3 parts:

*1. The Feature extractor (FE)*

The FE processes the $\boldsymbol{X}_i$ to extract $\boldsymbol{F}_i$. It starts with a one-to-one binary layer that acts as a binary feature selector, by multiplying each of its inputs by either zero or one. Adjusting the value of these weights during back-propagation is a difficult task, because their integer nature does not allow to calculate the gradient to be minimized during the training process. For this reason, the inventors used continuous weights constrained in the range [0, 1] for the calculation of the gradient, but rounded them to the nearest integer during the calculation of the prediction (i.e. the inference phase). To force this filter layer to sparsely select the features, they introduced a local *L1* regularization. However, the inventors empirically noticed that in this setup, the *L1* regularization pushes too many weights below 0.5 and, given that the gradient calculation does not see the rounding operation, it is difficult to increase their value afterwards. This phenomenon occurs especially in long training sessions. To solve this issue, they set a cut-off threshold t on the sum of the absolute values of the weights $w_k$ below wich *L1* regularization is no longer applied. In conclusion the selector layer, with respect to the rest of the FE, presents the following additional penalization:

$$L_{selec} = \max\left(\left(\sum_k |w_k|\right) - t, 0\right)$$

Apart from this selector layer, the rest of the FE is composed by fully connected layers (see Fig. 2) occasionally interspersed by dropout ones. In Figure 1, the symbol $\theta_{FE}$ is used to indicate all the parameters of the FE, including the selector's ones.

*2. The Predictor P*

P takes as input the $\boldsymbol{F}_i$, generated by the FE, and tries to predict the target output $y_i$, producing an estimated $\hat{y}_i$. The structure of P is composed by fully connected layers and its parameters are indicated with $\theta_P$.

*3. The confounders predictor C*

Similarly to P, C is composed by fully connected layers and tries to predict the confounders $c_i$ from $F_i$. Its parameters are indicated with $\theta_c$.

During the training, the DNN adjusts its parameters $\theta_{FE}$, $\theta_P$ and $\theta_C$ based on the cost function computed on a subset B of the available data, called batch. The parameter update happens in three rounds (see Fig. 1B) and involves three different loss functions and the regularization $L_{selec}$ described before.

The list of losses comprises:

1. A loss that minimizes the sum of the squared errors of the predictions made on the batch:

$$L_P = \sum_i^B (\hat{y}_i - y_i)^2$$

This loss is used to select the parameters that allow to correctly predict the task of interest (biological age or age of death).

2. A loss that minimizes the sum of the errors made in predicting the value of the confounders $c_{ij}$, where i and j are the indexes of the specific instance and confounder, respectively. The errors are computed differently for continuous confounders (using the root summed squared error) and categorical ones (using the cross-entropy):

$$L_{C1} = \sum_j L_{C,j} \rightarrow L_{C,j} = \begin{cases} \sqrt{\sum_i^B (\widehat{c_{ij}} - c_{ij})^2} \\ -\sum_i^B \sum_m^M c_{ijm} \log(c_{ijm}) \end{cases}$$

if $c_{ij}$ is continuous
if $c_{ij}$ is categorical This loss is used for training the confounder predictor C.

3. A loss that minimizes the sum across all the j confounders of the correlation between the vectors of the true and the predicted $j$-th confounder across the batch B.

$$L_{C2} = \sum_j corr_B^2(c_j, \widehat{c}_j)$$

This loss is used to remove the influence of the confounder variables from the vector $F_i$.

Note that the last two losses perform opposite tasks, using different metrics. This choice ensures that the output of **C** is as close as possible to the confounder values, so that when removing the dependency of the confounders from $F_i$ using a correlation metric, both linear and non-linear dependencies between $F_i$ and $c_i$ are destroyed.

The training starts with the of $\theta_{FE}$ and $\theta_P$ according to $L_P + \gamma L_{selec}$. Then, the parameters $\theta_c$ are updated according to $L_{C1}$, while keeping $\theta_{FE}$ (and thus $F_i$) stationary. Finally, $\theta_{FE}$ is updated again to remove the effects of confounders, using the loss $\gamma L_{selec} + \lambda L_{C2}$. These three steps, repeated for every batch, are equivalent to optimizing $\theta_{FE}$ with a saddle-point loss of the form:

$$L = L_P + \lambda L_{C2} + \gamma L_{selec}$$

Datasets composition

In this study three datasets have been analyzed, one for each of the analyses briefly described before:

Dataset I

It contains transcriptomic data extracted from different tissues and different strains of Notobranchius Furzeri (NF), with similar lifespan. These data have been used to perform the first analysis, i.e., the development of a pan-tissue and multi-strain TC. The age at which the samples have been collected are illustrated in Fig. 4A. More information on the dataset composition is collected in Table 1.

Dataset II

The transcriptomic data of this dataset belongs to a strain of NF, called GRZ, that has a lower lifespan with respect to the strains present in the dataset I. These data are used to study how the TC trained with the first dataset predicts the age of the GRZ fishes. The inventors expected to observe an overestimation of the predicted ages, because the GRZ strain has a faster aging process with respect to the strains used for the training. The GRZ ages tested are illustrated in Fig. 4B and some additional information on the dataset composition can be found in Table 2.

Dataset III

It contains the transcriptomic data of a longitudinal study, in which two fin samples of each fish have been acquired at 10 weeks and 20 weeks. From a biological point of view, except for the age of clip, all the data belong to a very homogeneous population: all the fishes are males, belongs to the same MZM strain and have been raised in the same housing condition. This data has been used for the third analysis: the development of a TT. In literature, life expectancy prediction is usually performed from the variation of an aging biomarker evaluated at two different points in lifetime, which is considered a good estimation of individual aging speed. In this study, instead, the inventors aimed to test the performance of a single-sample TT and verify whether there are some differences in estimating life expectancy at 10 weeks or at 20 weeks. In Fig. 4D the age of death distribution of this dataset is illustrated. Despite the impossibility to reliably estimate the shape of this distribution with the data of only 150 fishes, it is interesting to note that it appears much more uniform and symmetric with respect to the age of death distribution of humans, (see Ouellette et al., 2011, for comparison).

*Table* 1: Information available on dataset I other than the age of the samples

| Dataset I | | |
|---|---|---|
| Samples | 368 | |
| Fishes | 179 | |
| Tissue | Brain | 129 |
| | Liver | 55 |
| | Skin | 109 |
| | Muscle | 15 |
| | Fin | 60 |
| Strain | MZM | 252 |
| | MZMCS-0403 | 52 |
| | MZM-0403 | 44 |
| | line4020 | 20 |
| Sex | Male | 336 |
| | Female | 32 |
| Housing | Single | 313 |
| | Group | 55 |
| Batch | 14 different | batches |

*Table 2:* Information available on dataset II other than the age of the samples

| Dataset II | | |
|---|---|---|
| Samples | 147 | |
| Tissue | Brain | 61 |
| | Liver | 61 |
| | Skin | 25 |
| Strain | GRZ | |

*Table 3:* Information available on dataset III other than the length and weight of the fish at the clip time and its age of death.

| Dataset III | | |
|---|---|---|
| Samples | 300 | |
| Fishes | 150 | |
| Sample age | 10 weeks | 150 |
| | 20 weeks | 150 |
| Tissue | Fin | |
| Strain | MZM | |
| Sex | Male | |
| Batch | batch 1 | 90 |
| | batch2 | 60 |
| Hatch date | 11 dates between 2012 and 2014 | |

Dataset III also contains weight and length information of all the fishes at the moment of the two fin clips, which are obviously important biomarkers of a fish growth and consequently also of aging. In fact, a significant correlation between age of death and weight variation between the 10th and 20th week can be detected in this dataset (see Fig. 5B). The dataset reports also the hatch dates of the fishes (see Fig. 4C), that may constitute an important confounder, because fishes born in the same hatch or close time may have experienced similar external conditions. This hypothesis is empirically supported also by the weight distributions of the fishes born in different hatches, as shown in Fig. 5A.

Further information on dataset III is collected in Table 3.

Transcriptomic data normalization

Transcriptomic data of the three datasets have been separately normalized following this pipeline:

*1. Prefer.*
Counts related to genes whose length was inferior to 500 bp have been removed because not reliable.

*2. Correction for gene expression quantification from RNA-seq counts.*
The inventors applied the GeTMM (Smid et al., 2018) correction to each whole dataset. This method includes corrections for sequencing depth, gene length and total sample RNA output.

*3. Tissue-intersection filter.*
The inventors further filtered the genes to analyze, considering only the intersection of the gene lists whose expression exceeded 100 in almost 80 of the samples of a single tissue. This operation ensures that only genes that are significantly expressed in all the tissues under examination are considered.

*4. Single-element mathematical operations.*

First, as a common practice in gene expression analyses, the inventors applied a Log2-transformation to all the data. This operation non-linearly stretches the range of the values to a scale that better represents biologically relevant changes.

Second, before proceeding with the development of the TC and TT, the inventors divided each data point by the absolute maximum gene expression level m in the samples used for the training set. This operation is basically equal to dividing all the data by a constant, thus, it does not change the information encoded in each sample. Furthermore, by assessing the value of m exclusively on the training set, it was avoided to indirectly bring information from the validation and test sets into the training set.

It should be noted that despite it could be argued that a non-linear DNN, as the one used in this study, may autonomously learn to perform the two operations of step 4 in a data-driven way if necessary, implementing them before may reduce the training time and the amount of data necessary to reach good performance.

Finally, during the application of this pipeline to dataset II, the third step has been replaced with a filter that selected the same genes selected for the dataset I, because the inventors decided to have the same input genes in order to make the second analysis possible.

Analysis

Pan-tissue and multi-strain TC

For the development of the TC, the DNN structure described in the present Example and Figure 1, especially Fig. 1B, was used for setting the variables listed in Table 5 as confounders. The hyperparameters of the net have been chosen with a grid search varying the number of fully connected and dropout layers, the number of parameters, the learning rates of the three parts of the net and the $\gamma$ and $\lambda$ weights. In the optimized net, the FE is composed by 3 fully connected layers, C and P have the same structure and are composed by 2 fully connected layers. The vector $F_i$ extracted by FE is composed of 30 features. The total number of net parameters (excluding the selector) is around 7500.

The performance obtained with this net are summarized in Table 4, which shows that the errors committed on the test set are smaller than the ones on the training and validation sets: clearly the model did not overfit the data.

The correlation between the predicted ages and the true ones is illustrated in Fig. 6A. An analysis of the residuals depicted in Figure 6B-C shows that there is a mild systematic overestimation of age, that correlates with age. Despite its limited entity, this phenomenon is clearly not random. Probably the model had reached so small errors that improving the predictions was not convenient to minimize the cost-functions anymore and it focused on minimizing the regularization.

*Table 4:* Performance of the TC on dataset I. Note that fishes in the training set are different from the ones of the validation and test sets.

|  | Training | Validation | Test |
|---|---|---|---|
| Composition | 250 samples (150 fishes) | 66 samples (30 fishes) | 52 samples (29 fishes) |
| RMSE | 0.27 weeks | 0.26 weeks | 0.17 weeks |
| MAE | 0.21 weeks | 0.18 weeks | 0.12 weeks |

*Table 5:* Variables set as confounders in the development of TC.

| Variable | N classes |
|---|---|
| Batch | 14 |
| Strain | 4 |
| Tissue | 5 |
| Sex | 2 |
| Housing | 2 |
| Fish | 179 |

Residuals among possibly confounding categories (see Fig. 7) show that females' age seem to be overestimated with respect to males, but the inventors believe that this effect is mainly due to the higher age of female samples in the dataset (no female sample has been collected before 20 weeks of age). Some residual differences across the batches can be noted but considering the high number of batches and the low entity of these differences, they are probably caused by different age composition. To better investigate the effectiveness of the present example's strategy to avoid confounding effects, the point biserial correlation between the predicted age and the various confounders when adopting the adversarial learning, i.e. with $\lambda \neq 0$, and when switching it off, i.e. with $\lambda = 0$ was plotted in Fig 8. As it can be seen without adversarial learning the model would have learned a pattern based on sex, housing and strain, which are in fact the most biased confounders in dataset I. However, this framework allows to avoid learning from these variables. This suggests that all the previous works in which the training set was composed by an heterogenous set of data (e.g., using samples belonging to different tissues or analyzed in different batches or belonging to both females and males) and that did not implement any strategy to deal with confounding effect, may have biased predictions even if this was not explicitly reported. In this work instead, the use of the adversarial learning ensures that the algorithm has learned a generalizable pattern, exclusively based on how the gene expression depends on the aging process.

Overall, this experiment reports the best performing age estimations of a clock ever seen in prior art. This also proves that the information necessary to estimate the age of an individual is entirely contained in its transcriptome, suggesting that the errors of the previous clocks are caused by limitations of the algorithm and should not be used as an indication of abnormal aging speed.

TC application to a new strain with shorter lifespan

In this analysis the inventors applied the TC described in the previous section to dataset II, that is composed solely by samples taken from a strain with a shorter expected lifespan with respect to the ones used to train the predictor. As, expected the results revealed that the TC commits larger errors and systematically overestimates the age of GRZ samples, see Fig. 9. This suggests that GRZ strain has a faster aging process characterized by a different transcriptomic pattern, which explains the larger errors committed. Fitting the predicted ages with a linear regression (Fig 9A-B), an almost zero intercept and a positive slope was found, further confirming the hypothesis that GRZ fishes display a different but almost constant velocity of aging.

Overall, this experiment shows that the aging pattern learnt by the present algorithm is generalizable enough to be applied to an unseen strain with a different life expectancy. This is not trivial, and it was possible thanks to the use of the adversarial approach, that allowed to isolate a pattern independent from the strains used in the training set, and to the strategies dedicated to reducing overfitting. Finally, this experiment shows that the learnt pattern is sensitive to inherited differences in aging speed, while it remains unclear whether other clocks in prior art have this feature or simply identified a pattern based on the health state of the individuals.

Single-sample TT

For the optimization of the DNN structure for the development of a single-tissue TT, the inventors explored two configurations: the one already adopted for the TC and the same structure with the addition of some dropout layers in the FE. From this preliminary comparison it emerges clearly that this task requires the dropout layers to reduce overfitting. Two version of the TT have been developed one setting fish identifier, batch and hatch date as confounders only, the other one including also fish weight and length at the moment of sample acquisition (See Table 4). With these two TT versions, the inventors aimed to study how much the fish dimension information encoded in the transcriptome is essential to predict the number of remaining days of life.

Despite the few configurations explored for optimization, the results clearly show that developing a single-sample TT with significantly improved performances is possible (See Table 4). Furthermore, the dimension information seems of help to improve the prediction, but its removal does not prevent from learning anything about life expectancy (Fig 10). Fig 11 shows that for both the TT versions, the number of remaining days of life are overestimated for short-living fishes and underestimated for long-living ones. The overestimation maybe due to the fact that fishes with a very short lifespan, probably did not die exclusively because of an innate faster aging process, but with some external contributing factors, that may have occurred after sample acquisition. The underestimation instead may be tied to non-linear aging progression at higher ages. Finally, Fig. 12 shows that the prediction error does not depend on whether the data have been taken at 10 or 20 weeks of life of the fish. This is a new biological result because it has not been described before that the age of death would depend both on genetic and external factors, the latter being accumulated over time and thus being more informative later in life. This result suggests that lifespan variability is almost completely explained by the genetic predisposition of each fish.

A $R^2$ = 94% between the predicted and true time to death is an unprecedent results. This shows that the transcriptome information not only contains all the information necessary to predict the age of a sample but also to predict the time to death of an individual, and this information is present very early during adult life. This result would not have been possible without a short-living vertebrate such as the NF, which allowed the authors to collect clean and well annotated data, and without the use of a deep learning algorithm. In fact, previous attempts to face this problem with linear approaches obtained much inferior results, for example the method in (Lu, Ake T., et al. 2019) reached an $R^2$ = 40%.

**REFERENCES**

Vetter, Valentin Max, et al. "Relationship between five Epigenetic Clocks, Telomere Length and Functional Capacity as-sessed in Older Adults: Cross-sectional and Longitudinal Analyses." J Gerontol A Biol Sci Med Sci, Jan 15 2022;glab381 (2022).

Aramillo Irizar P, Schäuble S, et. al. Transcriptomic alterations during ageing reflect the shift from cancer to degenerative diseases in the elderly. Nat Commun. 2018 Jan 30;9(1):327. doi: 10.1038/s41467-017-02395-2. Erratum in: Nat Commun. 2019 May 31;10(1):2459.

Meyer, David H., and Björn Schumacher. "BiT age: A transcriptome-based aging clock near the theoretical limit of accuracy." Aging cell 20.3 (2021): e13320.

Terzibasi, Eva, Dario Riccardo Valenzano, and Alessandro Cellerino. "The short-lived fish Nothobranchius furzeri as a new model system for aging studies." Experimental gerontology 42.1-2 (2007): 81-89.

Adeli, Ehsan, et al. "Representation learning with statistical independence to mitigate bias." Proceedings of the IEEE/CVF Winter Conference on Applications of Computer Vision. 2021.

Trelin, Andrii, and Ales Prochazka. "Binary Stochastic Filtering: a Method for Neural Network Size Minimization and Su-pervised Feature Selection." arXiv preprint arXiv:1902.04510 (2019).

Lu, Ake T., et al. "DNA methylation GrimAge strongly predicts lifespan and healthspan." Aging (Albany NY) 11.2 (2019): 303.

Levine, Morgan E., et al. "An epigenetic biomarker of aging for lifespan and healthspan." Aging (Albany NY) 10.4 (2018): 573.

Ouellette, N. and Bourbeau, R. "Changes in the age-at-death distribution in four low mortality countries: A nonparametric approach". Demographic Research, 25:595-628, 2011.

Marcel Smid, Robert RJ Coebergh van den Braak, Harmen JG van de Werken, Job van Riet, Anne van Galen, Vanja de Weerd, Michelle van der Vlugt-Daane, Sandra I Bril, Zarina S Lalmahomed, Wigard P Kloosterman, et al. "Gene length corrected trimmed mean of m-values (getmm) processing of rna-seq data performs similarly in intersample analyses while improving intrasample comparisons". BMC bioinformatics, 19(1):1-13, 2018.

**Claims**

1. A method for predicting the lifespan of an individual, comprising

   i. providing biological input data of the subject,
   ii. providing a list of confounding variables,
   iii. predicting an age-related mortality or disease for the subject by analyzing the data with an algorithm,
   wherein the algorithm comprises a neural network, which is trained on at least one reference dataset comprising biological data of multiple reference subjects by applying:

   a) a selector layer to filter input data of i.,
   and
   b) an adversarial learning framework, that removes from the input data the information related to the confounding variables.

2. The method according to claim 1, wherein the selector assigns a weight between 0 and 1 to each variable by multiplying said variable with a number between 0 and 1, wherein a weight is not modified during the gradient calculation but is rounded to the nearest integer during the inference process.

3. The method according to claim 2, wherein a cut-L1 norm regularization is used to encourage the assignment of 0-weights by the selector,

   wherein a standard L1 norm regularization imposes the deep neural network to minimize the sum of the selector layer weights, and
   wherein said minimization is inactivated when the sum of the weights is below a threshold value.

4. The method according to claim 1, wherein the adversarial learning framework, is a neural network comprising three elements:

   I. a feature extractor (FE),
   II. a predictor (P), and
   III. a confounder predictor (C).

5. The method according to any one of the preceding claims, wherein

   the feature extractor (FE) is responsible for reducing the input information to a vector $\vec{F}$ with a lower dimensionality, whose elements are a non-linear combination of the inputs,
   the predictor (P), uses the output of FE to predict an age-related mortality or disease paired to the input data X , and
   the confounder predictor (C), uses the output of FE to predict a vector of at least one categorical and/or continuous confounder(s), which are associated to the input data X.

6. The method according to any one of the preceding claims, wherein the deep neural network is trained by adversarial machine learning by cyclical repetition of the following steps, comprising at least one repetition:

   a) optimizing the parameters of FE and P to minimize the prediction error of P,
   b) optimizing the parameter of C to minimize the error of C,
   c) optimizing the parameter of FE to maximize the error of C.

7. The method according to any one of the claims 5-6, wherein the at least one confounder(s) is selected from the group comprising gender, sample properties and technical variables relevant for acquiring the data.

8. The method according any one of the preceding claims wherein step iii. of predicting an age-related mortality or disease comprises predicting the time to death, a mortality risk and/or a risk of age-related disease of the subject.

9. The method according to any one of the preceding claims, wherein the biological data is selected from the group comprising genetic-, genomic-, proteomic-, metabolic-, immunological-, transcriptomic- or phenotypic-data or any combination thereof.

10. The method according to any one of the preceding claims, wherein the subject is a human, or an animal model, preferably a fish, a mouse or another vertebrate.

11. The method according to any one of the preceding claims, wherein the biological data of the subject is selected from the group comprising genome data, epigenome data, transcriptome data, proteome data, metabolome data or interactome data.

12. Use of the method according to any one of the preceding claims for predicting an age-related mortality or disease for the subject, preferably for predicting the time to death, the mortality risk and/or the risk of age-related disease for the subject.

13. Use of the method according to any one of the preceding claims for predicting the influence of a substance on the lifespan of a subject, wherein biological data, preferably according to any one of claims 9 and/or 11, of the subject are obtained at least after receiving treatment with said substance.

14. A software or computer program [product] for predicting the lifespan of a subject, wherein when said software or computer program product is executed the following steps are conducted:

  i. receiving biological data of the subject,
  ii. receiving a list of confounding variables,
  iii. predicting an age-related mortality or disease for the subject by analyzing the data with an algorithm, wherein the algorithm comprises a deep neural network, which is trained on at least one reference dataset comprising biological data of at least one reference subject by applying:

    a) a selector layer to select data variables,
    and
    b) an adversarial learning framework, that removes from the input data the information related to the confounding variables, and

  iv. transmitting and optionally displaying an output of the software or computer program product to a graphical user interface.

15. The software or computer program [product] according to claim 14, wherein the adversarial learning framework, is a neural network comprising three elements

  I. a feature extractor (FE),
  II. a predictor (P), and
  III. a confounder predictor (C).

16. A computer-readable storage device, comprising a software or computer program product according to the preceding claim.

**Fig. 1**

**Fig. 1 continued**

**Fig. 2**

input layer

hidden layer 1   hidden layer 2

output layer

**Fig. 3**

Fig. 4

**Fig. 5**

A

B

**Fig. 6**

A

o Training set
x Validation set
z Test set

B

o Training set
x Validation set
z Test set

C

Fig. 7

**Fig. 7 continued**

**Fig. 7 continued**

E

**Fig. 8**

**Fig. 9**

A

B

C

**Fig. 10**

A — Without weight and length as confounders

B — Using weight and length as confounders

**Fig. 11**

A

Without weight and length as confounders

B

With weight and length as confounders

**Fig. 12**

A

## Without weight and length as confounders

B

## With weight and length as confounders

Europäisches
Patentamt

European
Patent Office

Office européen
des brevets

# EUROPEAN SEARCH REPORT

Application Number

**EP 22 18 5439**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/234729 A1 (INSILICO MEDICINE IP LTD [CN]) 26 November 2020 (2020-11-26) <br> * the whole document * <br> * in particular * <br> * paragraph [0020] – paragraph [0053] * <br> * paragraph [0071] – paragraph [0076] * <br> * paragraph [0185] – paragraph [0198] * <br> ----- | 1-16 | INV. <br> G16H50/30 <br> G16H50/20 <br> G16B5/10 <br> G16B20/40 <br> G16B25/10 <br> G16B40/20 <br> G16B40/30 |
| | | | TECHNICAL FIELDS SEARCHED (IPC) |
| | | | G16B <br> G16H |

The present search report has been drawn up for all claims

| Place of search | Date of completion of the search | Examiner |
|---|---|---|
| Munich | 12 December 2022 | Rákossy, Z |

## ANNEX TO THE EUROPEAN SEARCH REPORT
## ON EUROPEAN PATENT APPLICATION NO.

**EP 22 18 5439**

This annex lists the patent family members relating to the patent documents cited in the above-mentioned European search report.
The members are as contained in the European Patent Office EDP file on
The European Patent Office is in no way liable for these particulars which are merely given for the purpose of information.

**12-12-2022**

| Patent document cited in search report | | Publication date | Patent family member(s) | | Publication date |
|---|---|---|---|---|---|
| WO 2020234729 | A1 | 26-11-2020 | CN 114450750 | A | 06-05-2022 |
| | | | EP 3970150 | A1 | 23-03-2022 |
| | | | WO 2020234729 | A1 | 26-11-2020 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VETTER, VALENTIN MAX et al.** Relationship between five Epigenetic Clocks, Telomere Length and Functional Capacity as-sessed in Older Adults: Cross-sectional and Longitudinal Analyses. *J Gerontol A Biol Sci Med Sci,* 15 January 2022, glab381
- **ARAMILLO IRIZAR P ; SCHÄUBLE S.** Transcriptomic alterations during ageing reflect the shift from cancer to degenerative diseases in the elderly. *Nat Commun.,* 30 January 2018, vol. 9 (1), 327
- **ERRATUM.** *Nat Commun.,* 31 May 2019, vol. 10 (1), 2459
- **MEYER, DAVID H. ; BJÖRN SCHUMACHER.** BiT age: A transcriptome-based aging clock near the theoretical limit of accuracy. *Aging cell,* 2021, vol. 20 (3), e13320
- **TERZIBASI, EVA ; DARIO RICCARDO VALENZANO ; ALESSANDRO CELLERINO.** The short-lived fish Nothobranchius furzeri as a new model system for aging studies. *Experimental gerontology,* 2007, vol. 42 (1-2), 81-89
- **ADELI, EHSAN et al.** Representation learning with statistical independence to mitigate bias. *Proceedings of the IEEE/CVF Winter Conference on Applications of Computer Vision,* 2021

- **TRELIN, ANDRII ; ALES PROCHAZKA.** Binary Stochastic Filtering: a Method for Neural Network Size Minimization and Su-pervised Feature Selection. *arXiv:1902.04510,* 2019
- **LU, AKE T. et al.** DNA methylation GrimAge strongly predicts lifespan and healthspan. *Aging (Albany NY),* 2019, vol. 11 (2), 303
- **LEVINE, MORGAN E. et al.** An epigenetic biomarker of aging for lifespan and healthspan. *Aging (Albany NY,* 2018, vol. 10 (4), 573
- **OUELLETTE, N. ; BOURBEAU, R.** Changes in the age-at-death distribution in four low mortality countries: A nonparametric approach. *Demographic Research,* 2011, vol. 25, 595-628
- **MARCEL SMID ; ROBERT RJ COEBERGH VAN DEN BRAAK ; HARMEN JG VAN DE WERKEN ; JOB VAN RIET ; ANNE VAN GALEN ; VANJA DE WEERD ; MICHELLE VAN DER VLUGT-DAANE ; SANDRA I BRIL ; ZARINA S LALMAHOMED ; WIGARD P KLOOSTERMAN et al.** Gene length corrected trimmed mean of m-values (getmm) processing of rna-seq data performs similarly in intersample analyses while improving intrasample comparisons. *BMC bioinformatics,* 2018, vol. 19 (1), 1-13